# EUROPEAN PATENT APPLICATION

(11) **EP 4 421 187 A2**
(43) Date of publication of application: **28.08.2024**
(21) Application number: 24172605.8
(22) Date of filing: 29.03.2021
(51) Int. Cl.: C12Q 1/6853

(54) **METHODS AND COMPOSITIONS FOR PREPARING NUCLEIC ACID LIBRARIES**

(30) Priority: 30.03.2020 US 202063001684 P
(62) Divisional of application: 21720109.4
(71) Applicant: Illumina, Inc., San Diego, CA 92122 (US)
(72) Inventor: CHRISTIANSEN, Lena, San Diego, 92122 (US); POKHOLOK, Dmitry K., San Diego, 92122 (US); STEEMERS, Frank J., San Diego, 92122 (US); PANTOJA, Rigo, San Diego, 92122 (US); CHU, Megan, San Diego, 92122 (US); IAVICOLI, Patrizia, San Diego, 92122 (US); CHANG, Weihua, San Diego, 92122 (US); BRODIN, Jeffrey, San Diego, 92122 (US); VERMAAS, Eric, San Diego, 92122 (US); THOMAS, Jerushah, San Diego, 92122 (US); ZHANG, Fan, San Diego, 92122 (US)
(74) Representative: Witte, Weller & Partner Patentanwälte mbB

(57) **Abstract**

Embodiments relate to the preparation of nucleic acid libraries. Some embodiments relate to the preparation of normalized nucleic acid libraries, such as libraries in which the amounts of amplified nucleic acids are substantially the same for different amounts of input nucleic acids. Some embodiments relate to preparation of indexed nucleic acid libraries with certain adaptors.

## Description

### CROSS-REFERENCE TO RELATED APPLICATIONS

This application claims priority to U.S. Prov. App. No. 63/001,684 filed March 30, 2020 entitled "METHOD AND COMPOSITIONS FOR PREPARING NORMALIZED NUCLEIC ACID LIBRARIES" which is incorporated herein by reference in its entirety.

### REFERENCE TO SEQUENCE LISTING

The present application is being filed along with a Sequence Listing in electronic format. The Sequence Listing is provided as a file entitled ILLINC450WOSEQLIST, created March 22, 2021, which is approximately 2 Kb in size. The information in the electronic format of the Sequence Listing is incorporated herein by reference in its entirety.

### FIELD OF THE INVENTION

Embodiments relate to the preparation of nucleic acid libraries. Some embodiments relate to the preparation of normalized nucleic acid libraries, such as libraries in which the amounts of amplified nucleic acids are substantially the same for different amounts of input nucleic acids. Some embodiments relate to preparation of indexed nucleic acid libraries with certain adaptors.

### BACKGROUND OF THE INVENTION

As common practice, biological samples used to prepare nucleic acid libraries for downstream analyses are homogeneously processed according to standardized assay protocols without regard to customized procedures for each sample. An important quality control (QC) measurement is nucleic acid concentration of nucleic acid libraries since performance of many modern nucleic acid analysis technologies is dependent on the nucleic acid concentration of input nucleic acids. Since such downstream analyses typically use expensive reagents and incur significant costs to perform, samples not meeting nucleic acid concentration requirements and/or other QC measurements are simply discarded on the assumption that the sample preparation was intrinsically unsuitable for the desired application.

For example, in next-generation sequencing (NGS) applications, also known as high-throughput sequencing, it is often desirable but logistically challenging to prepare nucleic acid libraries with substantially uniform DNA molar concentrations. This problem often arises in the preparation of nucleic acid libraries constructed from a plurality of heterogeneous biological samples, particularly when a desirable source biological sample is disproportionately underrepresented. This problem additionally arises when a target nucleic acid molecule is a relatively rarer and/or more unstable nucleic acid species when compared to even other nucleic acids that are derived from the same biological sample. It is challenging to create a nucleic acid library from sources such as a genome, with relatively equal representation of different regions of the genome.

### SUMMARY OF THE INVENTION

Some embodiments include a method of normalizing a nucleic acid library comprising target nucleic acids, comprising: (a) obtaining a substrate having a normalizing amount of capture probes attached thereto, wherein the capture probes comprise first amplification sites; (b) hybridizing a plurality of target nucleic acids to the capture probes; (c) extending the capture probes to obtain extended probes, wherein the extended probes comprise second amplification sites; and (d) amplifying the extended probes by hybridizing extension primers to the second amplification sites, wherein the amount of the extension primers is equal to or greater than the normalizing amount of capture probes, wherein the amplification is performed such that substantially all the capture probes are extended, thereby obtaining a normalizing amount of amplified target nucleic acids; wherein the first amplification sites and the extension primers are incapable of or are essentially incapable of hybridizing to one another.

In some embodiments, the first amplification sites and the extension primers are non-complementary to one another.

In some embodiments, the first amplification sites and the extension primers comprise non-complementary nucleotide sequences to one another.

In some embodiments, the first amplification sites comprise modified nucleotides that inhibit hybridization with the extension primers.

In some embodiments, the first amplification sites and the extension primers each lack the same type of nucleotide.

In some embodiments, the first amplification sites lack types of nucleotides complementary to one another, and each extension primer consists essentially of the same types of nucleotides as the first amplification site.

In some embodiments, the first amplification sites lack at least one type of nucleotide selected from adenine (A), cytosine (C), guanine (G), and thymine (T).

In some embodiments, the first amplification sites lack a combination of nucleotides selected from: adenine (A) and thymine (T); or guanine (G) and cytosine (C).

In some embodiments, the first amplification sites consist of a combination of nucleotides selected from: adenine (A) and guanine (G); adenine (A) and cytosine (C); cytosine (C) and thymine (T); or guanine (G) and thymine (T).

In some embodiments, the first amplification sites consist of a combination of adenine (A) and guanine (G) nucleotides.

In some embodiments, the extension primers are in solution.

In some embodiments, the capture probes comprise first indexes or first sequencing primer sites.

In some embodiments, the capture probes comprise first locus specific primers.

In some embodiments, the plurality of target nucleic acids hybridize to the first locus specific primers.

In some embodiments, extending the capture probes comprises ligating the first locus specific primers to the second locus specific primers.

In some embodiments, extending the capture probes comprises: (i) hybridizing second locus specific primers to the target nucleic acids; and (ii) ligating the first locus specific primers to the second locus specific primers.

In some embodiments, the second locus specific primers comprise the second amplification sites.

In some embodiments, the second locus specific primers comprise second indexes, or second sequencing primer sites.

Some embodiments also include: (iii) hybridizing extension oligonucleotides to the second locus specific primers; and (iv) extending the second locus specific primers with sequences complementary to the extension oligonucleotides by polymerase extension.

In some embodiments, the extension oligonucleotides comprise sites complementary to the second amplification sites, complementary to second indexes, or complementary to second sequencing primer sites.

In some embodiments, the target nucleic acids comprise sites complementary to the second amplification sites, and the extending comprises polymerase extension of the first locus specific primers with sequences complementary to the target nucleic acids.

Some embodiments also include preparing the target nucleic acids by adding adaptors to an end of the target nucleic acids, wherein the adaptors comprise sites complementary to the second amplification sites.

In some embodiments, adding adaptors comprises a tagmentation reaction.

In some embodiments, the substrate comprises a plurality of beads.

In some embodiments, the substrate comprises a flow cell.

In some embodiments, the amplification is performed under conditions such that the normalizing amount of capture probes limits the amount of amplification products.

Some embodiments also include sequencing the amplified target nucleic acids.

Some embodiments include a method of preparing a library of nucleic acids, comprising: (a) obtaining a substrate having a plurality of capture probes attached thereto, wherein the capture probes comprise first amplification sites and first locus specific primers; (b) hybridizing a plurality of target nucleic acids to the first locus specific primers; (c) hybridizing second locus specific primers to the target nucleic acids; (d) hybridizing the second locus specific primers to extension oligonucleotides, wherein the extension oligonucleotides comprise sites complementary to second amplification sites; and (e) extending the hybridized second locus specific primers to obtain a plurality of extended probes by: (i) ligating the first locus specific primers to the second locus specific primers, and (ii) extending the ligated second locus specific primers with sequences complementary to the extension oligonucleotides by polymerase extension.

In some embodiments, (i) comprises extending the first locus specific primers, and ligating the first locus specific primers to the second locus specific primers.

In some embodiments, the capture probes comprise first indexes, or first sequencing primer sites.

In some embodiments, the extension oligonucleotides comprise sites complementary to second indexes, or complementary to second sequencing primer sites.

In some embodiments, the substrate comprises a plurality of beads.

In some embodiments, the substrate comprises a flow cell.

Some embodiments also include obtaining a normalized amount of amplified target nucleic acids, comprising: amplifying the extended probes by hybridizing extension primers to the second amplification sites, wherein the substrate comprises a normalizing amount of the capture probes, and the amount of the extension primers is equal to or greater than the normalizing amount of capture probes.

In some embodiments, the extension primers are in solution.

In some embodiments, the amplification is performed under conditions such that the normalizing amount of capture probes limits the amount of amplification products.

Some embodiments also include sequencing the amplified target nucleic acids.

In some embodiments, the first amplification sites or second amplification sites comprise a P5 sequence, a complement of a P5 sequence, P7 sequence, or a complement of a P7 sequence.

In some embodiments, the first amplification sites and the extension primers are incapable of or are essentially incapable of hybridizing to one another.

In some embodiments, the first amplification sites and the extension primers are non-complementary to one another.

In some embodiments, the first amplification sites and the extension primers comprise non-complementary nucleotide sequences to one another.

In some embodiments, the first amplification sites comprise modified nucleotides that inhibit hybridization with the extension primers.

In some embodiments, the first amplification sites and the extension primers each lack the same type of nucleotide.

In some embodiments, the first amplification sites lack at least one type of nucleotide selected from adenine (A), cytosine (C), guanine (G), and thymine (T).

In some embodiments, the first amplification site lacks a combination of nucleotides selected from: adenine (A) and thymine (T); or guanine (G) and cytosine (C).

In some embodiments, the first amplification site consists of a combination of nucleotides selected from: adenine (A) and guanine (G); adenine (A) and cytosine (C); cytosine (C) and thymine (T); or guanine (G) and thymine (T).

In some embodiments, the first amplification site consists of a combination of adenine (A) and guanine (G) nucleotides.

Some embodiments include a method comprising: (a) obtaining a substrate comprising a plurality of capture probes and a plurality of extension primers attached thereto, wherein the capture probes comprise first amplification sites and first locus specific primers, and the extension primers are capable of hybridizing to second amplification sites, wherein the amount of the capture probes or the extension primers is a normalizing amount; (b) hybridizing a plurality of target nucleic acids to the first locus specific primers; (c) extending the capture probes to obtain extended probes, wherein the extended probes comprise second amplification sites; (e) amplifying the extended probes by hybridizing the extended probes to the extension primers, to obtain a normalized amount of amplified target nucleic acids.

In some embodiments, the normalizing amount limits the amount of amplified target nucleic acids.

In some embodiments, the capture probes comprise first indexes, or first sequencing primer sites.

In some embodiments, the extension oligonucleotides comprise sites complementary to second indexes, or complementary to second sequencing primer sites.

In some embodiments, the amplification is performed under conditions such that the normalizing amount of capture probes or the extension primers limits the amount of amplification products.

In some embodiments, the substrate comprises a plurality of beads.

In some embodiments, the substrate comprises a flow cell.

In some embodiments, the amplification comprises bridge amplification.

Some embodiments also include sequencing the amplified target nucleic acids.

In some embodiments, extending the capture probes comprises ligating the first locus specific primers to the second locus specific primers.

In some embodiments, extending the capture probes comprises: (i) hybridizing second locus specific primers to the target nucleic acids; and (ii) ligating the first locus specific primers to the second locus specific primers.

In some embodiments, the second locus specific primers comprise the second amplification sites.

In some embodiments, the second locus specific primers comprise second indexes or second sequencing primer sites.

Some embodiments also include: (iii) hybridizing extension oligonucleotides to the second locus specific primers; and (iv) extending the second locus specific primers with sequences complementary to the extension oligonucleotides by polymerase extension.

In some embodiments, the extension oligonucleotides comprise sites complementary to the second amplification sites, complementary to second indexes, or complementary to second sequencing primer sites.

In some embodiments, the target nucleic acids comprise sites complementary to the second amplification sites, and the extending comprises polymerase extension of the first locus specific primers with sequences complementary to the target nucleic acids.

Some embodiments also include preparing the target nucleic acids by adding adaptors to an end of the target nucleic acids, wherein the adaptors comprise sites complementary to the second amplification sites.

In some embodiments, adding adaptors comprises a tagmentation reaction.

Some embodiments also include determining the presence or absence of a variant in the target nucleic acid.

Some embodiments include a method of preparing an indexed nucleic acid library, comprising: (a) adding Y-adaptors to a plurality of target nucleic acids, wherein the Y-adaptors are added to first and second ends of each target nucleic acid, and each Y-adaptor comprises a first strand comprising a first primer binding site and a mosaic element, and a second strand comprising a second primer binding site and a complement to the mosaic element; and (b) hybridizing a first extension oligonucleotide to the second primer binding site, wherein the first extension oligonucleotide comprises a first index and a third primer binding site; and (c) extending the second strand comprising the second primer binding site, thereby adding the first index to the target nucleic acids.

In some embodiments, a 5' end of the first strand of the Y-adaptor is resistant to nuclease degradation.

In some embodiments, a 5' end of the first strand of the Y-adaptor comprises a phosphorothioate bond between two consecutive nucleotides.

In some embodiments, a 5' end of the second strand of the Y-adaptor is phosphorylated.

In some embodiments, (a) comprises contacting the plurality of target nucleic acids with a plurality of transposomes, wherein each transposome comprises a Y-adaptor and a transposase.

In some embodiments, the transposomes comprise dimers.

In some embodiments, the transposase comprises a Tn5 transposase.

In some embodiments, the transposomes are bound to a substrate.

In some embodiments, the substrate comprises a plurality of beads.

In some embodiments, the beads are magnetic.

In some embodiments, a 3' end of the first extension oligonucleotide is blocked.

In some embodiments, a 5' end of the first extension oligonucleotide is phosphorylated.

In some embodiments, the first extension oligonucleotide is bound to a bead.

Some embodiments also include cleaving the first extension oligonucleotide from the bead prior to (c).

In some embodiments, (c) comprises polymerase extension.

In some embodiments, (c) comprises extension with a ligase.

In some embodiments, extension with a ligase comprises: hybridizing a ligation oligonucleotide to the first extension oligonucleotide hybridized to the second primer binding site; and ligating the ligation oligonucleotide to the second strand comprising the second primer binding site.

In some embodiments, the ligation oligonucleotide comprises an additional index.

In some embodiments, the ligation oligonucleotide comprises an additional primer binding site.

Some embodiments also include removing the first extension oligonucleotide after step (c).

In some embodiments, the removing comprises an exonuclease treatment.

In some embodiments, the first extension oligonucleotide comprises uracil nucleotides, and the removing comprises degradation of the first extension oligonucleotide with a uracil-specific excision reagent (USER) enzyme.

Some embodiments also include amplifying the target nucleic acids comprising the first index.

In some embodiments, the amplification comprises hybridizing amplification primers to the first primer binding sites and to the third primer binding sites.

In some embodiments, the amplification comprises a PCR.

In some embodiments, the amplification comprises bridge amplification.

Some embodiments also include adding a second index to the target nucleic acids comprising the first index.

In some embodiments, adding the second index comprises: hybridizing a second extension oligonucleotide to the third primer binding site of the target nucleic acids comprising the first index, wherein the second extension oligonucleotide comprises the second index; and extending the second strand comprising the third primer binding site, thereby adding the second index to the target nucleic acids comprising the first index.

In some embodiments, a 3' end of the second extension oligonucleotide is blocked.

In some embodiments, a 5' end of the second extension oligonucleotide is phosphorylated.

In some embodiments, extending the second strand comprising the third primer binding site comprises polymerase extension.

In some embodiments, extending the second strand comprising the third primer binding site comprises extension with a ligase.

Some embodiments also include removing the second extension oligonucleotide after extending the third primer binding site.

Some embodiments also include adding a third index to the target nucleic acids comprising the second index.

Some embodiments also include adding an additional index to the target nucleic acids comprising the third index.

In some embodiments, the target nucleic acids comprise genomic DNA.

Some embodiments include a method of combinatorial indexing a plurality of target nucleic acids, comprising: (a) obtaining a pool of primary indexed nucleic acids, comprising: adding a first index to a plurality of subpopulations of target nucleic acids by extending the target nucleic acids with the first index, wherein a different first index is added to each subpopulation, and combining the subpopulations comprising the different first indexes to obtain the pool of primary indexed nucleic acids, wherein the first index is added to a subpopulation of target nucleic acids according to the method of any one of the foregoing methods; (b) splitting the pool into a plurality of subpopulations of primary indexed nucleic acids; (c) obtaining a pool of secondary indexed nucleic acids, comprising: adding a second index to the plurality of subpopulations of primary indexed nucleic acids by extending the primary indexed nucleic acids with the second index, wherein a different second index is added to each subpopulation, and combining the subpopulations comprising the different second indexes to obtain the pool of secondary indexed nucleic acids

Some embodiments also include repeating (b) and (c) and adding additional indexes to indexed subpopulations.

In some embodiments, adding a first index to a subpopulation of target nucleic acids is performed in a compartment selected from a well, a channel, or a droplet.

Some embodiments include a method of modifying a nucleic acid comprising: amplifying or extending the nucleic acid in the presence of a polymerase.

Some embodiments of any one of the foregoing methods include methods in which the amplifying and/or the extending is performed under conditions suitable to remove inorganic pyrophosphate (PPi), to inhibit pyrohosphorolysis, and/or inhibit formation of a Mg²⁺-PPi complex. In some embodiments, the PPi is soluble. In some embodiments, the amplifying and/or the extending is performed in the presence of an inorganic pyrophosphatase.

In some embodiments, a rate of achieving a yield of a product of the amplifying and/or the extending is increased in the presence of the inorganic pyrophosphatase compared to a rate of achieving a yield of a product of the amplifying and/or the extending in the absence of the inorganic pyrophosphatase. In some embodiments, the rate of achieving a yield of a product of the amplifying and/or the extending is increased by at least 2-fold.

In some embodiments, the amplifying and/or the extending is performed under isothermal conditions.

In some embodiments, the amplifying and/or the extending comprises performing a reaction selected from a PCR, a bridge amplification, a whole genome amplification, a loop-mediated isothermal amplification (LAMP), an amplification from nucleic acids obtained from a single cell, a sequencing by synthesis (SBS) reaction, and an exclusion amplification (ExAMp). In some embodiments, the amplifying and/or the extending comprises performing an amplification from nucleic acids obtained from a single cell. In some embodiments, the amplifying and/or the extending comprises performing a sequencing by synthesis (SBS) reaction. In some embodiments, the amplifying and/or the extending comprises performing a bridge amplification. In some embodiments, a sequencing reaction comprises the amplifying and/or the extending. The invention includes the following aspects defined in the following clauses which form part of the present description, which, however, do not represent claims, in accordance with the decision J15/88 of the Legal Board of Appeal of the European Patent Office.
1. A method of normalizing a nucleic acid library comprising target nucleic acids, comprising:
   (a) obtaining a substrate having a normalizing amount of capture probes attached thereto, wherein the capture probes comprise first amplification sites;
   (b) hybridizing a plurality of target nucleic acids to the capture probes;
   (c) extending the capture probes to obtain extended probes, wherein the extended probes comprise second amplification sites; and
   (d) amplifying the extended probes by hybridizing extension primers to the second amplification sites, wherein the amount of the extension primers is equal to or greater than the normalizing amount of capture probes, wherein the amplification is performed such that substantially all the capture probes are extended, thereby obtaining a normalizing amount of amplified target nucleic acids;
   wherein the first amplification sites and the extension primers are incapable of or are essentially incapable of hybridizing to one another.
2. The method of clause 1, wherein the first amplification sites and the extension primers are non-complementary to one another.
3. The method of clause 1, wherein the first amplification sites and the extension primers comprise non-complementary nucleotide sequences to one another.
4. The method of clause 1, wherein the first amplification sites comprise modified nucleotides that inhibit hybridization with the extension primers.
5. The method of clause 1, wherein the first amplification sites and the extension primers each lack the same type of nucleotide.
6. The method of clause 1, wherein the first amplification sites lack types of nucleotides complementary to one another, and each extension primer consists essentially of the same types of nucleotides as the first amplification site.
7. The method of clause 1, wherein the first amplification sites lack at least one type of nucleotide selected from adenine (A), cytosine (C), guanine (G), and thymine (T).
8. The method of clause 1, wherein the first amplification sites lack a combination of nucleotides selected from: adenine (A) and thymine (T); or guanine (G) and cytosine (C).
9. The method of clause 7 or 8, wherein the first amplification sites consist of a combination of nucleotides selected from: adenine (A) and guanine (G); adenine (A) and cytosine (C); cytosine (C) and thymine (T); or guanine (G) and thymine (T).
10. The method of any one of clauses 1-9, wherein the first amplification sites consist of a combination of adenine (A) and guanine (G) nucleotides.
11. The method of any one of clauses 1-10, wherein the extension primers are in solution.
12. The method of any one of clauses 1-11, wherein the capture probes comprise first indexes or first sequencing primer sites.
13. The method of any one of clauses 1-12, wherein the capture probes comprise first locus specific primers.
14. The method of clause 13, wherein the plurality of target nucleic acids hybridize to the first locus specific primers.
15. The method of clause 14, wherein extending the capture probes comprises ligating the first locus specific primers to the second locus specific primers.
16. The method of clause 15, wherein extending the capture probes comprises:
   (i) hybridizing second locus specific primers to the target nucleic acids; and
   (ii) ligating the first locus specific primers to the second locus specific primers.
17. The method of clause 16, wherein the second locus specific primers comprise the second amplification sites.
18. The method of clause 16 or 17, wherein the second locus specific primers comprise second indexes, or second sequencing primer sites.
19. The method of clause 16, further comprising:
   (iii) hybridizing extension oligonucleotides to the second locus specific primers; and
   (iv) extending the second locus specific primers with sequences complementary to the extension oligonucleotides by polymerase extension.
20. The method of clause 19, wherein the extension oligonucleotides comprise sites complementary to the second amplification sites, complementary to second indexes, or complementary to second sequencing primer sites.
21. The method of any one of clauses 16-20, wherein the target nucleic acids comprise sites complementary to the second amplification sites, and the extending comprises polymerase extension of the first locus specific primers with sequences complementary to the target nucleic acids.
22. The method of clause 21, further comprising preparing the target nucleic acids by adding adaptors to an end of the target nucleic acids, wherein the adaptors comprise sites complementary to the second amplification sites.
23. The method of clause 22, wherein adding adaptors comprises a tagmentation reaction.
24. The method of any one of clauses 1-23, wherein the substrate comprises a plurality of beads.
25. The method of any one of clauses 1-24, wherein the substrate comprises a flow cell.
26. The method of any one of clauses 1-25, wherein the amplification is performed under conditions such that the normalizing amount of capture probes limits the amount of amplification products.
27. The method of any one of clauses 1-26, further comprising sequencing the amplified target nucleic acids.
28. A method of preparing a library of nucleic acids, comprising:
   (a) obtaining a substrate having a plurality of capture probes attached thereto, wherein the capture probes comprise first amplification sites and first locus specific primers;
   (b) hybridizing a plurality of target nucleic acids to the first locus specific primers;
   (c) hybridizing second locus specific primers to the target nucleic acids;
   (d) hybridizing the second locus specific primers to extension oligonucleotides, wherein the extension oligonucleotides comprise sites complementary to second amplification sites; and
   (e) extending the hybridized second locus specific primers to obtain a plurality of extended probes by:
      (i) ligating the first locus specific primers to the second locus specific primers, and
      (ii) extending the ligated second locus specific primers with sequences complementary to the extension oligonucleotides by polymerase extension.
29. The method of clause 28, wherein (i) comprises extending the first locus specific primers, and ligating the first locus specific primers to the second locus specific primers.
30. The method of clauses 28 or 29, wherein the capture probes comprise first indexes, or first sequencing primer sites.
31. The method of any one of clauses 28-30, wherein the extension oligonucleotides comprise sites complementary to second indexes, or complementary to second sequencing primer sites.
32. The method of any one of clauses 28-31, wherein the substrate comprises a plurality of beads.
33. The method of any one of clauses 28-32, wherein the substrate comprises a flow cell.
34. The method of any one of clauses 28-33, further comprising obtaining a normalized amount of amplified target nucleic acids, comprising:
   amplifying the extended probes by hybridizing extension primers to the second amplification sites, wherein the substrate comprises a normalizing amount of the capture probes, and the amount of the extension primers is equal to or greater than the normalizing amount of capture probes.
35. The method of clause 34, wherein the extension primers are in solution.
36. The method of clauses 34 or 35, wherein the amplification is performed under conditions such that the normalizing amount of capture probes limits the amount of amplification products.
37. The method of any one of clauses 34-36, further comprising sequencing the amplified target nucleic acids.
38. The method of any one of clauses 28-37, wherein the first amplification sites or second amplification sites comprise a P5 sequence, a complement of a P5 sequence, P7 sequence, or a complement of a P7 sequence.
39. The method of any one of clauses 28-38, wherein the first amplification sites and the extension primers are incapable of or are essentially incapable of hybridizing to one another.
40. The method of clause 39, wherein the first amplification sites and the extension primers are non-complementary to one another.
41. The method of clause 39, wherein the first amplification sites and the extension primers comprise non-complementary nucleotide sequences to one another.
42. The method of clause 39, wherein the first amplification sites comprise modified nucleotides that inhibit hybridization with the extension primers.
43. The method of clause 39, wherein the first amplification sites and the extension primers each lack the same type of nucleotide.
44. The method of clause 39, wherein the first amplification sites lack at least one type of nucleotide selected from adenine (A), cytosine (C), guanine (G), and thymine (T).
45. The method of clause 39, wherein the first amplification site lacks a combination of nucleotides selected from: adenine (A) and thymine (T); or guanine (G) and cytosine (C).
46. The method of clauses 44 or 45, wherein the first amplification site consists of a combination of nucleotides selected from: adenine (A) and guanine (G); adenine (A) and cytosine (C); cytosine (C) and thymine (T); or guanine (G) and thymine (T).
47. The method of any one of clauses 44-46, wherein the first amplification site consists of a combination of adenine (A) and guanine (G) nucleotides.
48. A method of preparing a normalized nucleic acid library, comprising:
   (a) obtaining a substrate comprising a plurality of capture probes and a plurality of extension primers attached thereto, wherein the capture probes comprise first amplification sites and first locus specific primers, and the extension primers are capable of hybridizing to second amplification sites, wherein the amount of the capture probes or the extension primers is a normalizing amount;
   (b) hybridizing a plurality of target nucleic acids to the first locus specific primers;
   (c) extending the capture probes to obtain extended probes, wherein the extended probes comprise second amplification sites;
   (e) amplifying the extended probes by hybridizing the extended probes to the extension primers, to obtain a normalized amount of amplified target nucleic acids.
49. The method of clause 48, wherein the normalizing amount limits the amount of amplified target nucleic acids.
50. The method of clauses 48 or 49, wherein the capture probes comprise first indexes, or first sequencing primer sites.
51. The method of any one of clauses 48-50, wherein the extension oligonucleotides comprise sites complementary to second indexes, or complementary to second sequencing primer sites.
52. The method of any one of clauses 48-51, wherein the amplification is performed under conditions such that the normalizing amount of capture probes or the extension primers limits the amount of amplification products.
53. The method of any one of clauses 48-52, wherein the substrate comprises a plurality of beads.
54. The method of any one of clauses 48-53, wherein the substrate comprises a flow cell.
55. The method of any one of clauses 48-54, wherein the amplification comprises bridge amplification.
56. The method of any one of clauses 48-55, further comprising sequencing the amplified target nucleic acids.
57. The method of clause 48, wherein extending the capture probes comprises ligating the first locus specific primers to the second locus specific primers.
58. The method of clause 48, wherein extending the capture probes comprises:
   (i) hybridizing second locus specific primers to the target nucleic acids; and
   (ii) ligating the first locus specific primers to the second locus specific primers.
59. The method of clause 58, wherein the second locus specific primers comprise the second amplification sites.
60. The method of clauses 58 or 59, wherein the second locus specific primers comprise second indexes or second sequencing primer sites.
61. The method of clause 48, further comprising:
   (iii) hybridizing extension oligonucleotides to the second locus specific primers; and
   (iv) extending the second locus specific primers with sequences complementary to the extension oligonucleotides by polymerase extension.
62. The method of clause 61, wherein the extension oligonucleotides comprise sites complementary to the second amplification sites, complementary to second indexes, or complementary to second sequencing primer sites.
63. The method of clauses 61 or 62, wherein the target nucleic acids comprise sites complementary to the second amplification sites, and the extending comprises polymerase extension of the first locus specific primers with sequences complementary to the target nucleic acids.
64. The method of clause 63, further comprising preparing the target nucleic acids by adding adaptors to an end of the target nucleic acids, wherein the adaptors comprise sites complementary to the second amplification sites.
65. The method of clause 64, wherein adding adaptors comprises a tagmentation reaction.
66. The method of any one of clauses 48-65, further comprising determining the presence or absence of a variant in the target nucleic acid.
67. A method of preparing an indexed nucleic acid library, comprising:
   (a) adding Y-adaptors to a plurality of target nucleic acids, wherein the Y-adaptors are added to first and second ends of each target nucleic acid, and each Y-adaptor comprises a first strand comprising a first primer binding site and a mosaic element, and a second strand comprising a second primer binding site and a complement to the mosaic element; and
   (b) hybridizing a first extension oligonucleotide to the second primer binding site, wherein the first extension oligonucleotide comprises a first index and a third primer binding site; and
   (c) extending the second strand comprising the second primer binding site, thereby adding the first index to the target nucleic acids.
68. The method of clause 67, wherein a 5' end of the first strand of the Y-adaptor is resistant to nuclease degradation.
69. The method of clauses 67 or 68, wherein a 5' end of the first strand of the Y-adaptor comprises a phosphorothioate bond between two consecutive nucleotides.
70. The method of any one of clauses 67-69, wherein a 5' end of the second strand of the Y-adaptor is phosphorylated.
71. The method of any one of clauses 67-70, wherein (a) comprises contacting the plurality of target nucleic acids with a plurality of transposomes, wherein each transposome comprises a Y-adaptor and a transposase.
72. The method of clause 71, wherein the transposomes comprise dimers.
73. The method of clause 71 or 72, wherein the transposase comprises a Tn5 transposase.
74. The method of clause 71-73, wherein the transposomes are bound to a substrate.
75. The method of clause 74, wherein the substrate comprises a plurality of beads.
76. The method of clause 75, wherein the beads are magnetic.
77. The method of any one of clauses 67-76, wherein a 3' end of the first extension oligonucleotide is blocked.
78. The method of any one of clauses 67-77, wherein a 5' end of the first extension oligonucleotide is phosphorylated.
79. The method of any one of clauses 67-78, wherein the first extension oligonucleotide is bound to a bead.
80. The method of clause 79, further comprising cleaving the first extension oligonucleotide from the bead prior to (c).
81. The method of any one of clauses 67-80, wherein (c) comprises polymerase extension.
82. The method of any one of clauses 67-80, wherein (c) comprises extension with a ligase.
83. The method of clause 82, wherein extension with a ligase comprises:
   hybridizing a ligation oligonucleotide to the first extension oligonucleotide hybridized to the second primer binding site; and
   ligating the ligation oligonucleotide to the second strand comprising the second primer binding site.
84. The method of clause 83, wherein the ligation oligonucleotide comprises an additional index.
85. The method of clause 83, wherein the ligation oligonucleotide comprises an additional primer binding site.
86. The method of any one of clauses 67-85, further comprising removing the first extension oligonucleotide after step (c).
87. The method of clause 86, wherein the removing comprises an exonuclease treatment.
88. The method of clause 87, wherein the first extension oligonucleotide comprises uracil nucleotides, and the removing comprises degradation of the first extension oligonucleotide with a uracil-specific excision reagent (USER) enzyme.
89. The method of any one of clauses 67-88, further comprising amplifying the target nucleic acids comprising the first index.
90. The method of clause 89, wherein the amplification comprises hybridizing amplification primers to the first primer binding sites and to the third primer binding sites.
91. The method of clauses 89 or 90, wherein the amplification comprises a PCR.
92. The method of clauses 89 or 90, wherein the amplification comprises bridge amplification.
93. The method of any one of clauses 67-92, further comprising adding a second index to the target nucleic acids comprising the first index.
94. The method of clause 93, wherein adding the second index comprises:
   hybridizing a second extension oligonucleotide to the third primer binding site of the target nucleic acids comprising the first index, wherein the second extension oligonucleotide comprises the second index; and
   extending the second strand comprising the third primer binding site, thereby adding the second index to the target nucleic acids comprising the first index.
95. The method of clause 94, wherein a 3' end of the second extension oligonucleotide is blocked.
96. The method of clause 94 or 95, wherein a 5' end of the second extension oligonucleotide is phosphorylated.
97. The method of any one of clauses 94-96, wherein extending the second strand comprising the third primer binding site comprises polymerase extension.
98. The method of any one of clauses 94-96, wherein extending the second strand comprising the third primer binding site comprises extension with a ligase.
99. The method of any one of clauses 94-98, further comprising removing the second extension oligonucleotide after extending the third primer binding site.
100. The method of any one of clauses 94-99, further comprising adding a third index to the target nucleic acids comprising the second index.
101. The method of clause 100, further comprising adding an additional index to the target nucleic acids comprising the third index.
102. The method of any one of clauses 67-101, wherein the target nucleic acids comprise genomic DNA.
103. A method of combinatorial indexing a plurality of target nucleic acids, comprising:
   (a) obtaining a pool of primary indexed nucleic acids, comprising:
      adding a first index to a plurality of subpopulations of target nucleic acids by extending the target nucleic acids with the first index, wherein a different first index is added to each subpopulation, and
      combining the subpopulations comprising the different first indexes to obtain the pool of primary indexed nucleic acids;
   (b) splitting the pool into a plurality of subpopulations of primary indexed nucleic acids;
   (c) obtaining a pool of secondary indexed nucleic acids, comprising:
      adding a second index to the plurality of subpopulations of primary indexed nucleic acids by extending the primary indexed nucleic acids with the second index, wherein a different second index is added to each subpopulation, and
      combining the subpopulations comprising the different second indexes to obtain the pool of secondary indexed nucleic acids.
104. The method of clause 103, further comprising repeating (b) and (c) and adding additional indexes to indexed subpopulations.
105. The method of clauses 103 or 104, wherein adding a first index to a subpopulation of target nucleic acids is performed in a compartment selected from a well, a channel, or a droplet.
106. The method of any one of clauses 1-106, wherein the amplifying and/or the extending is performed under conditions suitable to remove inorganic pyrophosphate (PPi), to inhibit pyrohosphorolysis, and/or to inhibit formation of a Mg²⁺-PPi complex.
107. A method of modifying a nucleic acid comprising:
   amplifying or extending the nucleic acid in the presence of a polymerase, wherein the amplifying and/or the extending is performed under conditions suitable to remove inorganic pyrophosphate (PPi), to inhibit pyrohosphorolysis, and/or to inhibit formation of a Mg²⁺-PPi complex.
108. The method of clause 106 or 107, wherein the PPi is soluble.
109. The method of any one of clauses 1-108, wherein the amplifying and/or the extending is performed in the presence of an inorganic pyrophosphatase.
110. The method of clause 109, wherein a rate of achieving a yield of a product of the amplifying and/or the extending is increased in the presence of the inorganic pyrophosphatase compared to a rate of achieving a yield of a product of the amplifying and/or the extending in the absence of the inorganic pyrophosphatase.
111. The method of clause 110, wherein the rate of achieving a yield of a product of the amplifying and/or the extending is increased by at least 2-fold.
112. The method of any one of clauses 1-111, wherein the amplifying and/or the extending is performed under isothermal conditions.
113. The method of any one of clauses 1-112, wherein the amplifying and/or the extending comprises performing a reaction selected from a PCR, a bridge amplification, a whole genome amplification, a loop-mediated isothermal amplification (LAMP), an amplification from nucleic acids obtained from a single cell, a sequencing by synthesis (SBS) reaction, and an exclusion amplification (ExAMp).
114. The method of any one of clauses 1-113, wherein the amplifying and/or the extending comprises performing an amplification from nucleic acids obtained from a single cell.
115. The method of any one of clauses 1-113, wherein the amplifying and/or the extending comprises performing a sequencing by synthesis (SBS) reaction.
116. The method of any one of clauses 1-113, wherein the amplifying and/or the extending comprises performing a bridge amplification.
117. The method of any one of clauses 1-113, wherein a sequencing reaction comprises the amplifying and/or the extending.

### BRIEF DESCRIPTION OF THE DRAWINGS

FIG. 1 (panel A) depicts an embodiment of a workflow for normalizing amplification products on a bead. FIG. 1 (panel B) depicts alternative amplification steps in a workflow for normalizing a nucleic acid library on a bead.
FIG. 2 depicts an embodiment of a workflow for preparing a nucleic acid library in which genomic DNA is tagmented with an indexed transposome, and capture probes are extended with sequences complementary to the indexed transposome.
FIG. 3 depicts an embodiment of a workflow for preparing a nucleic acid library in which genomic DNA is tagmented, and capture probes are extended by ligation to an extension primer.
FIG. 4A depicts an embodiment of a workflow for preparing a nucleic acid library in which genomic DNA is tagmented with a transposome containing at T7 promoter, fragments are amplified by *in vitro* transcription, amplified RNA fragments hybridized to capture probes and the capture probes are extended by ligation to an extension primer.
FIG. 4B depicts an embodiment of a workflow for preparing a nucleic acid library in which genomic DNA is tagmented with a transposome containing at T7 promoter, adaptors are added during a reverse transcription (RT) extension, and extension products hybridized to capture probes on a flow cell.
FIG. 5 depicts an embodiment of a workflow for preparing a nucleic acid library in which genomic DNA is amplified, capture probes containing P5 sequences are extended by ligation to an extension primer, and the extension primer is extended with sequences complementary to an extension oligonucleotide.
FIG. 6 depicts an embodiment of a workflow for preparing a nucleic acid library in which genomic DNA is amplified, capture probes containing GGA sequences are extended by ligation to an extension primer, and the extension primer is extended with sequences complementary to an extension oligonucleotide.
FIG. 7 depicts an embodiment of normalization of amplification products on a bead with capture probes and extension probes each including the same types of non-complementary nucleotides.
FIG. 8 depicts a bead with capture probes containing P5 sequences hybridized to a target nucleic acid containing P7' sequences with extension primers containing P7 sequences in solution, and a bead with capture probes containing GGA sequences hybridized to a target nucleic acid containing AAG' sequences with extension primers containing AAG sequences in solution.
FIG. 9 is a photograph of a gel which shows that on-bead normalization using an AAG/GGA primer system yields less on-bead dimers than a P5/P7 primer system.
FIG. 10 is a photograph of a gel which shows that on-bead normalization using an AAG/GGA primer system yields substantially similar levels of normalized amplified products between high and low levels of initial input DNA, and a P5/P7 primer system yields different levels of purportedly normalized amplified products between high and low levels of initial input DNA.
FIG. 11 is a line graph comparing various amounts of input DNA using either an AAG/GGA primer system or a P5/P7 primer system to obtain relative amounts of output DNA.
FIG. 12 is a photograph of a gel showing various amounts of input DNA using either an AAG/GGA primer system or a P5/P7 primer system in which target nucleic acids are hybridized either overnight or for 1 hour.
FIG. 13 is a photograph of a gel showing various amounts of input DNA using either an AAG/GGA primer system or a P5/P7 primer system in which extended captures were amplified (+ExAMP), or not amplified (-ExAMP).
FIG. 14 is a schematic of an embodiment for single level indexing by hybridization-extension in solution.
FIG. 15 is a schematic of an embodiment for single level indexing by hybridization-extension on beads.
FIG. 16 is a schematic of an embodiment for multiple level indexing by hybridization-extension on beads.
FIG. 17 is a schematic of an embodiment for multiple level indexing by hybridization-extension in solution.
FIG. 18 is a schematic of an embodiment for three level indexing by multiple hybridizations-single extension/ligation in solution.
FIG. 19 is a schematic of an embodiment for single level indexing by hybridization-extension in droplets.
FIG. 20 is a schematic of an embodiment for single level indexing by hybridization in droplets with bulk extension.
FIG. 21 is a schematic of an embodiment in which a sample indexing nucleotide is extended and a gap between locus specific enrichment oligonucleotides is sealed in a single post enrichment step.
FIG. 22 is a graph of total DNA yield for amplification reactions performed in the presence or absence of iPPase (IPP) and sampled at incubation times of 0.5 hour, 1 hour, 2 hours, and 3 hours.
FIG. 23 is a graph of fluorescence intensity for amplification reactions performed with a 60K Infinium EX beadchip and in the presence or absence of iPPase (IPP) and sampled at incubation times of 0.5 hour, 1 hour, 2 hours, and 3 hours. For each time, the left and right columns are mean Xraw or Yraw channel fluorescent intensity values, respectively.
   Note that Xraw and Yraw intensities are greater for formulations that contain IPP at 30 min and 1 h.
FIG. 24 is a graph of call rate for amplification reactions performed in the presence or absence of iPPase (IPP) and sampled at incubation times of 0.5 hour, 1 hour, 2 hours, and 3 hours.

### DETAILED DESCRIPTION

Embodiments relate to the preparation of nucleic acid libraries. Some embodiments relate to the preparation of normalized nucleic acid libraries, such as libraries in which the amounts of (amplified) nucleic acids are substantially the same. Some embodiments relate to preparation of indexed nucleic acid libraries with certain adaptors.

Some embodiments relate to preparing normalized nucleic acid libraries having substantially similar amounts of target nucleic acids. In some such embodiments prepared normalized nucleic acid libraries having substantially similar amounts of amplified nucleic acids. In some embodiments, input nucleic acids are hybridized to capture probes attached to beads, the capture probes comprising first amplification sites lacking types of nucleotides complementary to one another. In some embodiments, the first amplification sites lack a single type nucleotide. In some embodiment the first amplification sites lack a combination of nucleotides selected from: adenine (A) and thymine (T); or guanine (G) and cytosine (C). In some embodiments, the first amplification sites consist of a combination of nucleotides selected from: adenine (A) and guanine (G); adenine (A) and cytosine (C); cytosine (C) and thymine (T); or guanine (G) and thymine (T). In some embodiments, the first amplification sites consist of a combination of adenine (A) and guanine (G) nucleotides. In some embodiments, the first amplification site can consist of one type of nucleotide.

The capture probe can be extended with sequences complementary to the hybridized input nucleic acid. In some embodiments, the capture probe is extended by polymerization. In some embodiments, the capture probe is extended by ligation of the capture probe with an oligonucleotide complementary to the hybridized input nucleic acid. In some embodiments, the capture probe is extended by polymerization and ligation. The extended capture probe can include a second amplification site and/or sample index.

The extended capture probe can be amplified on the bead by hybridizing extension primers in solution to the second amplification sites. In some embodiments, the amount of the extension primers is equal to or greater than a normalizing amount of capture probes. In some embodiments, the extension primers consists essentially of the same types of nucleotides as the first amplification site. In some such embodiments, the extension primers and the capture probes do not include complementary nucleotides, and interactions between the extension primers and capture probes are reduced, such as the formation of primer-dimers. The hybridized extension primers can be extended, and the products hybridized to non-extended capture probes on the beads. The extended capture probes are amplified such that substantially all the capture probes are extended, thereby obtaining a normalizing amount of amplified target nucleic acids.

Some embodiments relate to preparing a library of nucleic acids in which an adaptor is added by extending a target nucleic acid. In some embodiments, a target nucleic acid is hybridized to a capture probe comprising a first locus specific primer. The capture probe can be extended by hybridizing the hybridized target nucleic acid to a second locus specific primer. In some embodiments, the first and second locus specific primers can be ligated, thereby extending the capture probe. In some embodiments, the second locus specific primer can be extended by hybridizing an extension primer to the second locus specific primer, and extending the second locus specific primer with sequences complementary to the extension primer. In some embodiments, a sample index is introduced through an extension step.

### Certain definitions

As used herein, "hybridization", can refer to the ability of nucleic acid molecules to join via complementary base strand pairing. Such hybridization may occur when nucleic acid molecules are contacted under appropriate conditions and/or circumstances. As used herein, two nucleic acid molecules are said to be capable of specifically hybridizing to one another if the two molecules are capable of forming an anti-parallel, double-stranded nucleic acid structure. A nucleic acid molecule is said to be the "complement" of another nucleic acid molecule if they exhibit complete complementarity. As used herein, nucleic acid molecules are said to exhibit "complete complementarity" when every nucleotide of one of the molecules is complementary to its base pairing partner nucleotide of the other. Two molecules are said to be "minimally complementary" if they can hybridize to one another with sufficient stability to permit them to remain annealed to one another under at least conventional "low-stringency" conditions. In some instances, the molecules are said to be "complementary" if they can hybridize to one another with sufficient stability to permit them to remain annealed to one another under conventional "high-stringency" conditions. Nucleic acid molecules that hybridize to other nucleic acid molecules, *e.g.,* at least under low stringency conditions are said to be "hybridizable cognates" of the other nucleic acid molecules. Conventional stringency conditions are described by Sambrook et al., Molecular Cloning, A Laboratory Handbook, Cold Spring Harbor Laboratory Press, 1989), and by Haymes et al. In: Nucleic Acid Hybridization, A Practical Approach, IRL Press, Washington, D.C. (1985), each of which is herein incorporated by reference in its entirety, and for the disclosure discussed herein. Departures from complete complementarity are therefore permissible in some embodiments, as long as such departures do not completely preclude the capacity of the molecules to form a double-stranded structure. Thus, in order for a nucleic acid molecule or fragment thereof of the present disclosure to serve as a primer or probe in some embodiments it needs only be sufficiently complementary in sequence to be able to form a stable double-stranded structure under the particular solvent and salt concentrations employed. In some embodiments, nucleic acids disclosed herein are fully complementary to their targets.

As used herein, "nucleic acid molecule" and "polynucleotide" are used interchangeably herein, and refer to both RNA and DNA molecules, including nucleic acid molecules comprising cDNA, genomic DNA, synthetic DNA, and DNA or RNA molecules containing nucleic acid analogs. Nucleic acid molecules can have any three-dimensional structure. A nucleic acid molecule can be double-stranded or single-stranded (*e.g.,* a sense strand or an antisense strand). Non-limiting examples of nucleic acid molecules include genes, gene fragments, exons, introns, messenger RNA (mRNA), transfer RNA, ribosomal RNA, siRNA, micro-RNA, tracrRNAs, crRNAs, guide RNAs, ribozymes, cDNA, recombinant polynucleotides, branched polynucleotides, nucleic acid probes and nucleic acid primers. A nucleic acid molecule may contain unconventional or modified nucleotides. The terms "polynucleotide sequence" and "nucleic acid sequence" as used herein interchangeably refer to the sequence of a polynucleotide molecule. The nomenclature for nucleotide bases as set forth in 37 CFR 91. 822 is used herein.

As used herein, the term a "nucleic acid sample" refers to a collection of nucleic acid molecules. In some embodiments, the nucleic acid sample is from a single biological source, e.g. one individual or one tissue sample, and in other embodiments the nucleic acid sample is a pooled sample, e.g., containing nucleic acids from more than one organism, individual or tissue."

As used herein, "nucleic acid sample" encompasses "nucleic acid library" which, as used herein, includes a nucleic acid library that has been prepared by any method known in the art. In some embodiments, providing the nucleic acid library includes the steps required for preparing the library, for example, including the process of incorporating one or more nucleic acid samples into a vector-based collection, such as by ligation into a vector and transformation of a host. In some embodiments, providing a nucleic acid library includes the process of incorporating a nucleic acid sample into a non-vector-based collection, such as by ligation to adaptors. In some embodiments, the adaptors can anneal to PCR primers to facilitate amplification by PCR or can be universal primer regions such as, for example, sequencing tail adaptors. In some embodiments, the adaptors can be universal sequencing adaptors.

As used herein, "substantially" has its ordinary meaning as read in light of the specification, and can mean, for example, at least 60%, at least 70%, at least 80%, at least 85%, at least 90%, at least 95%, at least 96%, at least 97%, at least 98%, or at least 99%

### Normalizing amplification products

Some embodiments relate to preparing nucleic acid libraries having normalized amounts of amplified products. For example, the amounts of certain amplified products obtained from different target nucleic acids can be substantially the same between the amplified products. In some embodiments, the nucleic acid amounts in normalized nucleic acid libraries vary by less than about 30%, 20%, 10%, 5%, 3%, 2%, or 1%. In some embodiments, the nucleic acid amounts in normalized nucleic acid libraries vary by less than about 2-fold, 3-fold, 4-fold, or 5-fold. In some embodiments, pooled nucleic acid libraries are generated in which the amount of constituent nucleic acids in the resultant pooled nucleic acid libraries are at substantially similar amounts regardless of the amount of input nucleic acids. In some embodiments, the amounts of constituent nucleic acids in a pooled nucleic acid libraries vary by less than about 30%, 20%, 10%, 5%, 3%, 2%, or 1%. In some embodiments, the amounts of constituent nucleic acids in a pooled nucleic acid libraries vary by less than about 2-fold, 3-fold, 4-fold, or 5-fold.

Some embodiments of preparing a normalized nucleic acid library can include providing amplification conditions in which an amplification primer is provided in a limited amount, and other amplification reagents are provided in non-limiting amounts. In some such embodiments, the amount of the amplification products are limited according to the amount of the limited amplification primer. In some such embodiments, more than one amplification reaction can be performed, and each amplification reaction can result in an amount of amplification product which is substantially the same as the other amplification reaction(s).

In some embodiments, a substrate can be provided which includes a plurality of capture probes and a plurality of extension primers attached to the substrate. In some embodiments, a substrate comprises a solid support. In some embodiments, a substrate is a planar substrate. In some embodiments, a planar substrate includes discrete sites, such as wells. In some embodiments, a substrate can include a plurality of beads. In some embodiments, the capture probes can include first amplification sites. An amplification site can include a binding site for an amplification primer. In some embodiments, the extension primers can include sites capable of hybridizing to second amplification sites. In some embodiments, the capture probes, or the extension primers can be provided in a normalizing amount such that amplification of a target nucleic acid from the first and second amplification primers results in a normalized amount of amplification product. For example, the amount of the capture probes can be provided at an amount less than or equal to the extension primers, such that the amount of amplification products is limited by the amount of the capture probes; or the amount of the extension primers can be provided at an amount less than or equal to the capture probes, such that the amount of amplification products is limited by the amount of the extension primers.

In some embodiments, a nucleic acid sample can be hybridized to the capture probes, the capture probes can be extended to generate extended probes. In some embodiments, the extended probes can include a second amplification site capable of hybridizing to the extension primers. In some embodiments, the extended probes can be amplified by hybridizing the extended probes to the extension probes, the extension probes can be extended, and subsequent products hybridized to the capture probes and extension primers. In some embodiments, the amplification can include bridge amplification.

Some embodiments of normalizing a nucleic acid library comprising target nucleic acids can include obtaining a substrate having a normalizing amount of capture probes attached to the substrate, and a plurality of extension primers. In some embodiments, the substrate comprises a plurality of beads. In some embodiments, the capture probes comprise first amplification sites, and are incapable of or are essentially incapable of hybridizing to the extension primers. In some embodiments, the first amplification sites and the extension primers are non-complementary to one another. In some embodiments, the first amplification sites and the extension primers comprise non-complementary nucleotide sequences to one another. In some embodiments, the first amplification sites comprise modified nucleotides that inhibit hybridization with the extension primers. In some embodiments, the first amplification sites and the extension primers each lack the same type of nucleotide. In some embodiments, the capture probes comprise first amplification sites lacking types of nucleotides complementary to one another. In some embodiments, the first amplification sites lack a combination of nucleotides selected from: adenine (A) and thymine (T); or guanine (G) and cytosine (C). In some embodiments, the first amplification sites consist of a combination of nucleotides selected from: adenine (A) and guanine (G); adenine (A) and cytosine (C); cytosine (C) and thymine (T); or guanine (G) and thymine (T). In some embodiments, the first amplification sites consist of a combination of adenine (A) and guanine (G) nucleotides. In some embodiments, the primers can contain pseudo-complementary bases or bases and modifications that prevent dimer formation.

In some embodiments, the extension primers comprise sites capable of hybridizing to second amplification sites. In some embodiments, the extension primers consist essentially of the same types of nucleotides as the first amplification site. In some embodiments, the extension primers consist of the same types of nucleotides as the first amplification site. In some embodiments, the extension primers are in solution.

Some embodiments include hybridizing a plurality of target nucleic acids to the capture probes, and extending the capture probes to obtain extended probes. In some embodiments, the extended probes can be amplified by hybridizing the extended probes to the extension probes, the extended probes can be extended, and subsequent products hybridized to the capture probes and extension primers. In some embodiments, the amplification can include bridge amplification. In some embodiments, the amount of the extension primers is equal to or greater than the normalizing amount of capture probes, and the amplification is performed such that substantially all the capture probes are extended, to obtain a normalizing amount of amplified target nucleic acids.

In some embodiments, the DNA is amplified using whole genome amplification, hybridized to capture probes, formation of amplifiable targeted libraries, limited amplification with normalization, and sequencing of the normalized targeted sequencing library.

In some embodiments, a step is included for the analysis of the targeted sequencing library. For example, sequencing can include the capture probe and genomic DNA for the analysis of single nucleotide polymorphisms and variants. Sequencing of the capture probe can identify the targeted region, a variant, and sample index or barcode. In some embodiments, analysis does not include the alignment of the sequencing reads to a reference genome.

In some embodiments, the capture probes can include a first index, and/or the extension primer can include a sequence complementary to a second index. An index can include a sequence that can identify the source of a nucleic acid. For example, an index can identify the source of a target nucleic acid to a certain sample. In some embodiments, an index can identify the source of an amplification product of a target nucleic acid to a certain sample of nucleic acids. In some embodiments, an index can comprise less than 50, 40, 30, 20, 10, or 5 consecutive nucleotides, or a number of nucleotides between any two of the foregoing numbers.

In some embodiments, the capture probes can include a first sequencing primer site, and/or the extension primer can include a sequence complementary to a second sequencing primer site. A sequencing primer site can include a binding site for a sequencing primer. Examples of a sequencing primer site include a nucleic acids having the sequence P5, P7, or complements therefore.
Primer P5 AAT GAT ACG GCG ACC ACC GA (SEQ ID NO: 1)
Primer P7 CAA GCA GAA GAC GGC ATA CGA GAT (SEQ ID NO: 2)

In some embodiments, the capture probes can include a first locus specific primer. The first locus specific primer can include a nucleic acid sequence capable of binding to a first locus binding site in a target nucleic acid. In some embodiments, the capture probes can be extended by hybridizing a target nucleic acid to the first locus specific primer of the capture probe, and hybridizing a second locus specific primer to the target nucleic acid. In some embodiments, the first locus specific primer and second locus specific primer bind at locations on the target nucleic acid adjacent to one another. In some embodiments, the locations that the first locus specific primer and second locus specific primer bind on the target nucleic acid are less than 200, 100, 50, 40, 30, 20, 10 5, 1 consecutive nucleotides apart on the target nucleic acid, or any number of nucleotides apart on the target nucleic acid between any two of the foregoing numbers. In some embodiments, extending the capture probe can include ligating the first locus specific primer to the second locus specific primer. In some embodiments, extending the capture probe can include extending the first locus specific primer with a polymerase, and ligating the first locus specific primer to the second locus specific primer. In some embodiments, extending the capture probe can include extending the first locus specific primer with a polymerase.

In some embodiments, the second locus specific primers can include a second amplification site. In some embodiments, the second locus specific primers can include a second index. In some embodiments, the second locus specific primers can include a second sequencing primer site.

In some embodiments, an adaptor can be added to the second locus specific primer. The adaptor can include a second amplification site, a second index, and/or a second sequencing primer site. In some embodiments, the adaptor can be added by ligation or extension and ligation to the second locus specific primer. In some embodiments, an extension oligonucleotide can be hybridized to the second locus specific primer. The extension oligonucleotide can include sequences complementary to a second amplification site, a second index, and/or a second sequencing primer site. The second locus specific primer can be extended with sequences complementary to the extension oligonucleotide, and thereby include a second amplification site, a second index, and/or a second sequencing primer site. In some embodiments, a target nucleic acid is hybridized to a first locus specific primer and a second locus specific primer, and an extension oligonucleotide is hybridized to the second locus specific primer. In some embodiments, the target nucleic is hybridized to the first locus specific primer and the second locus specific primer, at the same time as the extension oligonucleotide is hybridized to the second locus specific primer. In some embodiments, the target nucleic is hybridized to the first locus specific primer and the second locus specific primer, prior to the extension oligonucleotide being hybridized to the second locus specific primer. In some embodiments, the target nucleic is hybridized to the first locus specific primer and the second locus specific primer, after the extension oligonucleotide being hybridized to the second locus specific primer. In some embodiments, the first locus specific primer is extended and joined to the second locus specific primer use of a polymerase and/or a ligase, the second locus specific primer is extended with a polymerase.

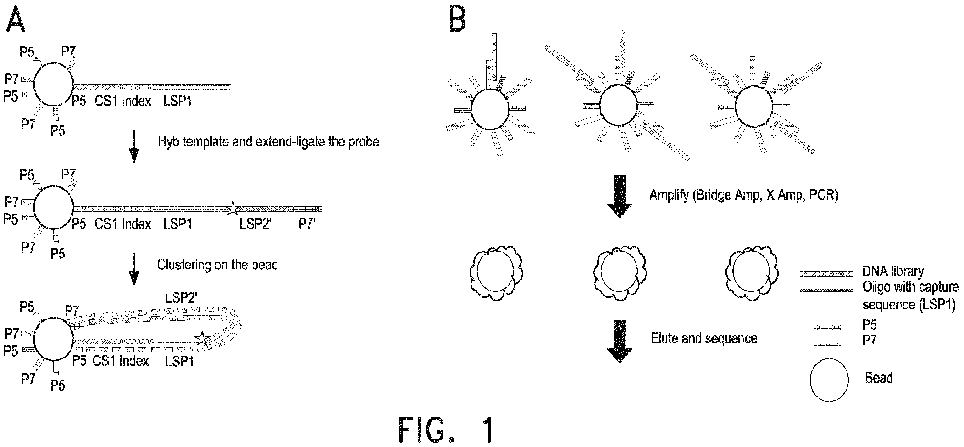

An example embodiment is illustrated in FIG. 1 (panel A) in which a capture probe is attached to a bead, the capture probe contains an amplification site (P5), a first sequencing primer site (CS1), an index, and a first locus specific probe (LSP1) designed one base upstream from a single nucleotide polymorphism (SNP) or insertion/deletion of interest. The beads are also grafted with a high concentration of extension primers containing sequencing adapters, such as P5 or P7. Of course, it should be realized that other sequencing adapters are contemplated. The capture probe is extended by hybridization to a target nucleic acid, and ligation with an oligonucleotide containing a second locus specific probe (LSP2) and sequence complementary to a P7 sequence (P7'). The extended capture probe is amplified on the bead. FIG. 1 (panel B) summarizes alternative amplification steps including bridge amplification, isothermal recombinase assisted amplification, or any other kind of clonal amplification. The density of the sequencing adapters on each bead surface determines the concentration of the final library after completion of the clustering reaction. Clustering time is optimized to reach full utilization of P5/P7 primers on the bead surface, regardless of initial target capture-extension efficiency. Later, amplified libraries are removed from beads, differentially indexed samples are pooled, clustered, and sequenced on an appropriate platform depending on assay plexity. FIG. 1 (panel B) illustrates three beads with different amounts of target nucleic acids hybridized to capture probes on the beads, and amplification on the beads results in normalized amounts of amplification products. By selecting a predetermined amount or density of sequencing adapters on the bead surface, one can alter the final concentration of a particular amplicon as its amplified on that bead.

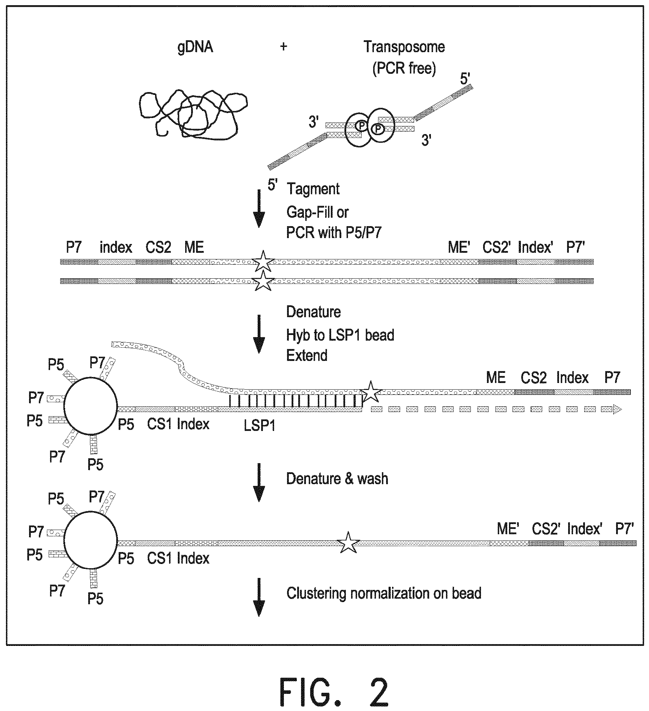

An example embodiment is depicted in FIG. 2 in which genomic DNA (gDNA) is tagmented with a transposome. The tagmentation reaction fragments the gDNA and adds adaptors to the DNA fragments. The adaptors include P7 sequences, a first index, and a common primer site (CS2). The ends of the fragments are gap-filed, and the fragments are amplified by PCR. The amplified fragments are hybridized with capture probes attached to beads. The capture probes include a P5 sequence, a common primer site (CS1), a second index, and a first locus specific primer (LSP1). The LSP1 is designed to bind immediately upstream from a single nucleotide polymorphism (star) of interest. Extension primers containing P7 and/or P5 sequences are attached to the beads. Unbound library is washed by stringent washes, and a locus specific probe is extended by high fidelity DNA polymerase to copy the SNP, downstream region and second sequencing adapter with index (CS2'_Index'_P7'). After the removal of the target DNA strand, the extended strand is subjected to normalization by clustering on the same bead, as depicted in FIG 1.

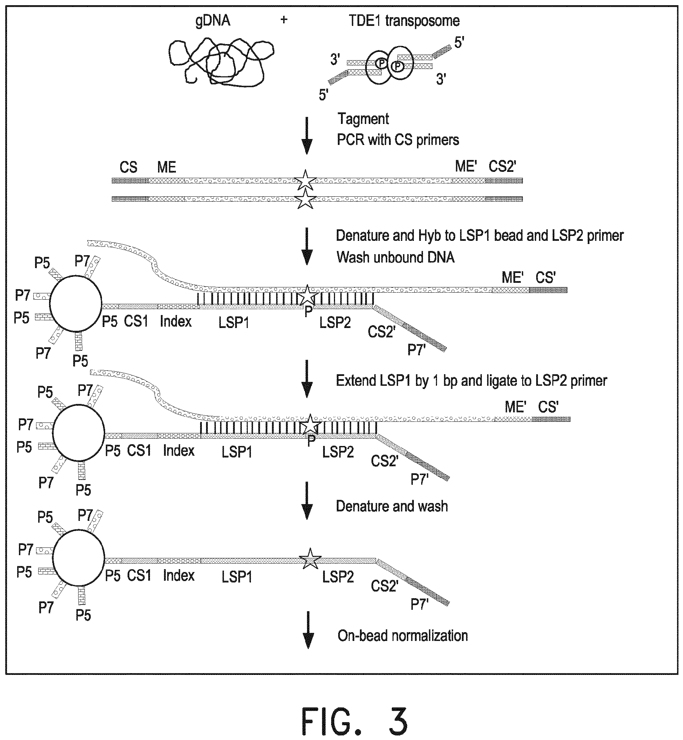

Another example embodiment is depicted in FIG. 3 in which gDNA is tagmented with a TDE1 transposome containing minimal adapter sequences. The tagmentation reaction fragments the gDNA, and adds minimal adaptors to the ends of the fragments, the adaptors containing mosaic element (ME) and common sequence (CS), and complements thereof. After tagmentation and gap-fill, the initial library is pre-amplified by PCR via the CS sites. The denatured library is hybridized to a capture probe attached to a bead, the capture probe containing a P5 sequence, a first common sequence (CS1), an index, and a first locus specific probe (LSP1). The LSP1 is designed to bind to target nucleic acids immediately upstream from a SNP of interest (star). An extension primer containing a 5' phosphorylated end, a second locus specific primer (LSP2), a complement to a second common sequence (CS2') and complement to a P7 sequence (P7') is hybridized one or a few bases downstream from the SNP. Unbound library and excess of the extension primer are washed by stringent washes, and LSP1 of the capture probe is extended over the SNP, and ligated to the downstream LSP2 of the extension primer. The resulting library strand, which now contains sequencing adapters, is washed and clustered on the same bead. Alternatively, input DNA can be tagmented with TSM containing the sequence of T7 or SP6 promoter and amplified by *in vitro* transcription (IVT).

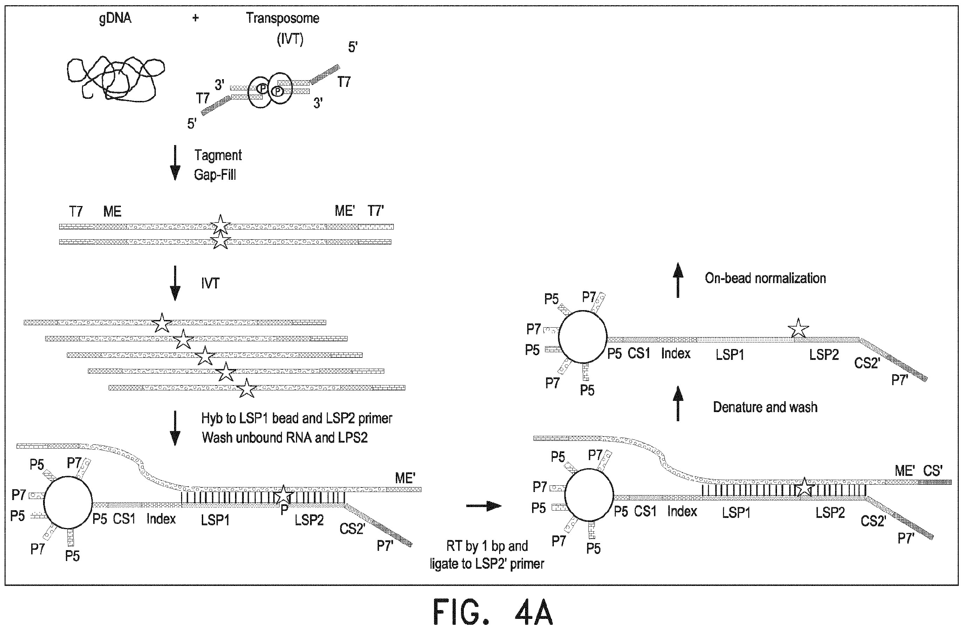

Yet another example embodiment is depicted in FIG. 4A in which gDNA is tagmented with transposome containing T7 sequences. The tagmentation reaction fragments the gDNA and adds adaptors to the ends of the fragments. The adaptors contain T7 and ME sequences, and complements thereof. The fragments are amplified by *in vitro* transcription to obtain a plurality of RNA. The RNA is hybridized to capture probe attached to a bead, the capture probe containing a P5 sequence, a first common sequence (CS1), an index, and a first locus specific probe (LSP1). The LSP1 is designed to bind to target nucleic acids immediately upstream from a SNP of interest (star). An extension primer containing a 5' phosphorylated end, a second locus specific primer (LSP2), a complement to a second common sequence (CS2') and complement to a P7 sequence (P7') is hybridized one or a few bases downstream from the SNP. The capture probe is extended with a reverse transcriptase, and the extended capture probe ligated to the extension primer. The resulting library strand, which now contains sequencing adapters, is washed and clustered on the same bead.

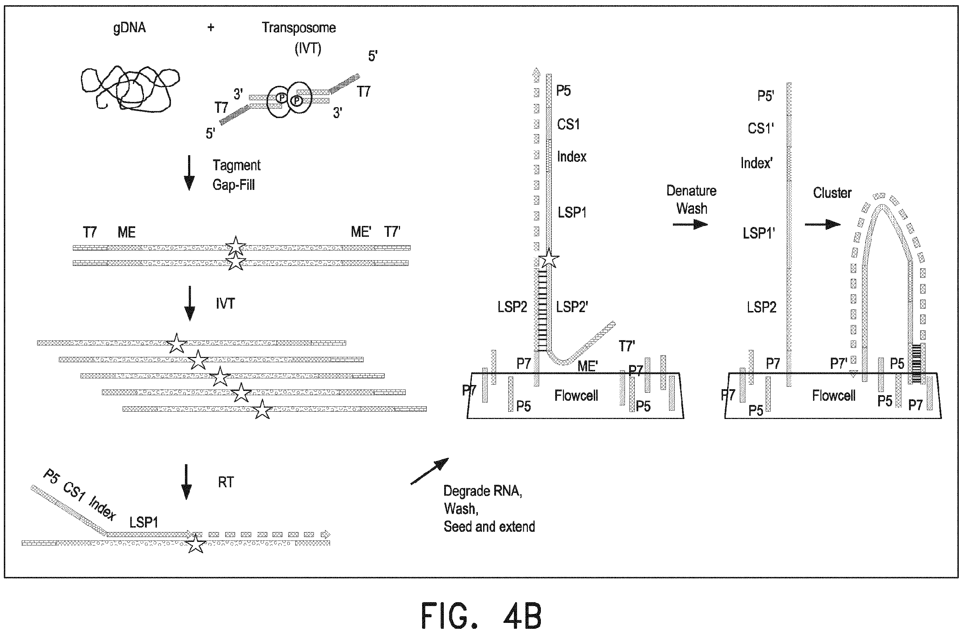

Another example embodiments is depicted in FIG. 4B in which gDNA is tagmented with a transposome containing T7 sequences. The tagmentation reaction fragments the gDNA and adds adaptors to the ends of the fragments. The adaptors contain T7 and ME sequences, and complements thereof. The fragments are amplified by *in vitro* transcription to obtain a plurality of RNA. The RNA is hybridized to an oligonucleotide containing a P5 sequence, a first common sequence (CS1), an index, and a first locus specific probe (LSP1). The LSP1 is designed to bind to target nucleic acids immediately upstream from a SNP of interest (star). The hybridized oligonucleotide is extended by reverse transcription (RT). The extension products are hybridized to capture probes attached to a flowcell via a region in the extended products that binds to a second locus specific primer (LSP2). The capture probe contains a P7 sequence and the second LSP2. The capture probe is extended, and the extended capture probes amplified by solid phase amplification, such as cluster amplification, for example bridge amplification.

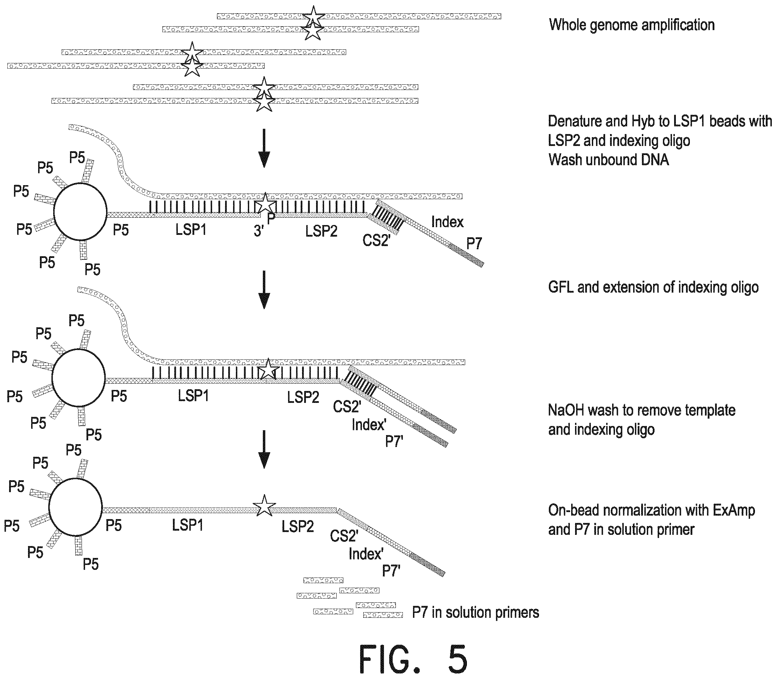

One other example embodiment is depicted in FIG. 5 in which amplified target nucleic acids containing a SNP of interest (star) are hybridized to capture probes attached to beads. Amplification can include linear amplification, or exponential amplification. Examples of amplification include whole genome amplification, and targeted amplification. The capture probes contain a P5 sequence and a first locus specific probe (LSP1). Extension primers are hybridized to the hybridized target nucleic acids, and extension oligonucleotides are hybridized to the extension primers. The extension primers contain a second locus specific primer (LSP2), and a second common sequence (CS2). The extension oligonucleotides hybridize to the CS2 region of the extension primers, and contain an index and a P7 sequence. The LSP1 of the capture probe is ligated to the LSP2 of the extension primer, and the extension primer is extended to include sequences complementary to the extension oligonucleotide. The hybridized target nucleic acid is removed. The extended capture probe is amplified via P5 primers attached to the beads, and P7 primers in solution. Amplification results in a normalized amount of amplification products because amplification is limited by the amount of P5 primers attached to the beads.

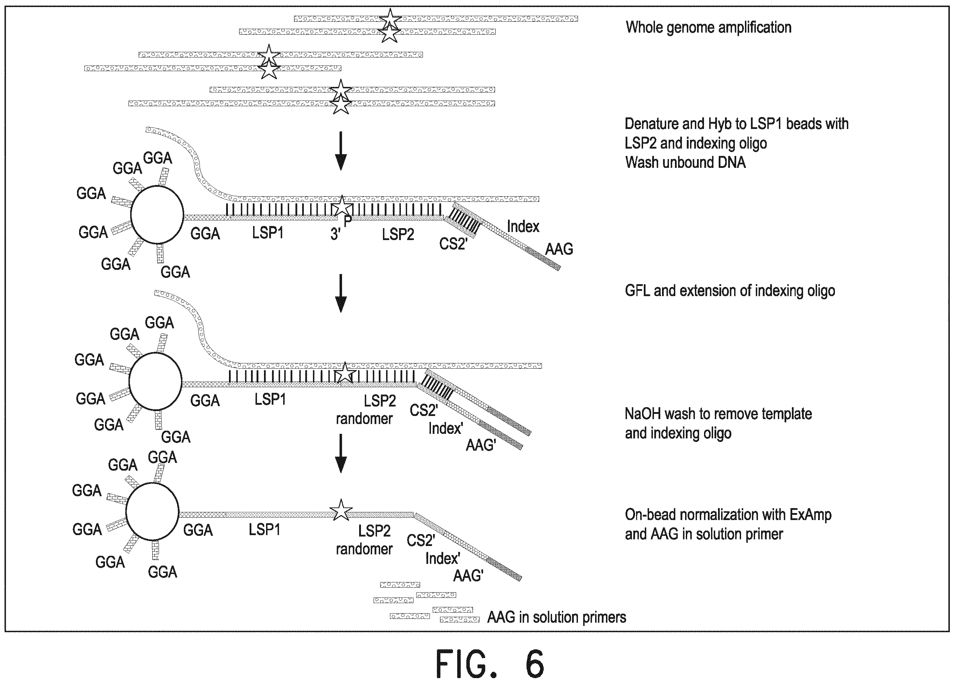

An example embodiment is also depicted in FIG. 6 in which amplified target nucleic acids containing a SNP of interest (star) are hybridized to capture probes attached to beads. The capture probes contain a GGA sequence and a first locus specific probe (LSP1). Extension primers are hybridized to the hybridized target nucleic acids, and extension oligonucleotides are hybridized to the extension primers. The extension primers contain a second locus specific primer (LSP2), and a second common sequence (CS2). The extension oligonucleotides hybridize to the CS2 region of the extension primers, and contain an index and an AAG sequence. The LSP1 of the capture probe is ligated to the LSP2 of the extension primer, and the extension primer is extended to include sequences complementary to the extension oligonucleotide. The hybridized target nucleic acid is removed. The extended capture probe is amplified via GGA primers attached to the beads, and AAG primers in solution. Amplification results in a normalized amount of amplification products because amplification is limited by the amount of GGA primers attached to the beads.

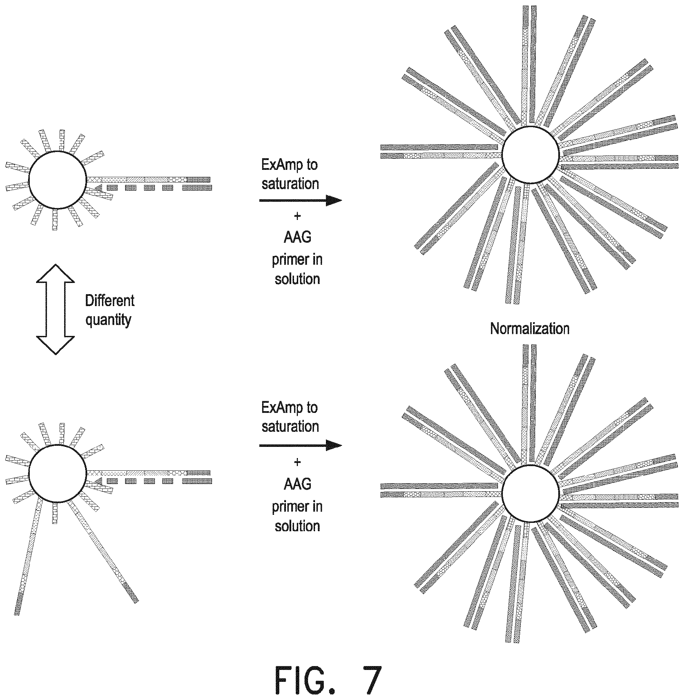

Differing amounts of input nucleic acids can result in substantially similar amounts of amplified products. As depicted in FIG. 7, different amounts of target nucleic acids are hybridized to capture probes attached to beads, and the capture probes are extended to generate different amounts of extended capture probes (left side of FIG. 7). The extended capture probes are amplified via first primers attached to the beads and second primers in solution. The amount of the amplified products is limited by the amount of first primers attached to the beads. Therefore, the amplification results in substantially similar amounts of amplified products even though the amounts of initial input nucleic acids was different.

In some embodiments, the target nucleic acid includes sequences complementary to the second amplification sites. In some embodiments, the target nucleic acid include sequences complementary to a second index and/or a second sequencing primer. In some such embodiments, a capture probe containing a first locus specific primer can be extended by hybridizing the target nucleic acid to the first locus specific primer, and extending the first locus specific primer with a polymerase to incorporate sequences complementary to the target nucleic acid, such as a second amplification site, a second index, and/or a second sequencing primer. In some embodiments, a target nucleic acid can be prepared by added adaptors that include sequences complementary to a second amplification sites, a second index and/or a second sequencing primer. In some embodiments, the target nucleic acid can be prepared by a tagmentation reaction. For example, an input nucleic acid comprising a target nucleic acid can be contacted with a plurality of transposomes. The transposomes can fragment the input nucleic acid, and attach adaptors to the ends of the nucleic acid fragments. Examples of tagmentation reactions are disclosed in U.S. Pat No. 9,040,256, which is incorporated by reference in its entirety.

In some embodiments, prepared nucleic acid libraries or pooled libraries can be suitable for downstream analytical applications, including sequencing applications utilizing techniques such as next-generation sequencing (NGS) and related methodologies such as genotyping-by-sequencing (GBS). For example, the first locus specific primer and second locus specific primer can bind to the target nucleic acid at locations which have single nucleotide polymorphism SNP, insertion, deletion, or duplication between the two binding sites. The amplified target nucleic acids can be sequenced and the SNP, insertion, deletion, or duplication can be identified.

In some embodiments, amplification products can be separated from a solid support, such as a bead. Generally, any suitable method for removing nucleic acids from the solid support can be used, such as use of enzymes, and/or changes in temperature and/or pH. In some embodiments, the amplification products are separated from the solid support by elution. In some embodiments, the amplification products are eluted in a heated buffer. In some embodiments where streptavidin is included in solid phase support and the nucleic acids are biotinylated to facilitate binding of the nucleic acids to the solid support, the nucleic acid amplification products can be separated from the solid support via by heated avidin-biotin cleavage.

In some embodiments, nucleic acid amplification is performed under substantially isothermal conditions. The optimal temperature for amplification varies and may for example depend on primer characteristics, such as sequence length, melting temperature, and choice of polymerase. In some embodiments, the amplification temperature is lower than 60 degrees Celsius, such as lower than 50 degrees Celsius, lower than 45 degrees Celsius, such as, 42, 38, 35, 30, 25, or 20 degrees Celsius. In some preferred embodiments, the amplification temperature is about 38 degrees Celsius. In some embodiments, isothermal amplification can be performed by using kinetic exclusion amplification (KEA), also referred to as exclusion amplification (Ex-Amp).

In some embodiments, an amplified/normalized nucleic acid library can be constructed by reacting an amplification reagent to produce a plurality of amplification sites that each includes a substantially clonal population of amplicons from an individual target nucleic acid that has seeded the site. In some embodiments, the amplification reaction proceeds until a sufficient number of amplicons are generated to fill the capacity of the respective amplification site. Filling an already seeded site to capacity in this way inhibits target nucleic acids from landing and amplifying at the site thereby producing a clonal population of amplicons at the site. In some embodiments, apparent clonality can be achieved even if an amplification site is not filled to capacity prior to a second target nucleic acid arriving at the site. Under some conditions, amplification of a first target nucleic acid can proceed to a point that a sufficient number of copies are made to effectively outcompete or overwhelm production of copies from a second target nucleic acid that is transported to the site. For example in an embodiment that uses a bridge amplification process on a circular feature (*e.g*., bead) that is smaller than 500 nm in diameter, it has been determined that after 14 cycles of exponential amplification for a first target nucleic acid, contamination from a second target nucleic acid at the same site will produce an insufficient number of contaminating amplicons to adversely impact sequencing-by-synthesis analysis on an Illumina sequencing platform.

In some embodiments, kinetic exclusion can occur when a process occurs at a sufficiently rapid rate to effectively exclude another event or process from occurring. For example, a solution of beads having universal primers where the beads are randomly seeded with target nucleic acids in a solution and copies of the target nucleic acids are generated in an amplification process to fill each of the beads to capacity. In accordance with the kinetic exclusion, the seeding and amplification processes can proceed simultaneously under conditions where the amplification rate exceeds the seeding rate. As such, the relatively rapid rate at which copies are made on a particular bead that has been seeded by a first target nucleic acid will effectively exclude a second nucleic acid from seeding that particular bead for amplification. Kinetic exclusion amplification methods can be performed as described in detail in the disclosure of U.S. Application Pub. No. 2013/0338042, which is incorporated herein by reference in its entirety.

In some embodiments, kinetic exclusion can exploit a relatively slow rate for initiating amplification, such as a slow rate of making a first copy of a target nucleic acid compared to a relatively rapid rate for making subsequent copies of the target nucleic acid, or of the first copy of the target nucleic acid. In some embodiments, kinetic exclusion occurs due to the relatively slow rate of target nucleic acid seeding, such as relatively slow diffusion or transport compared to the relatively rapid rate at which amplification occurs to fill the site, such as bead or other site on a solid substrate with copies of the nucleic acid seed. In another embodiment, kinetic exclusion can occur due to a delay in the formation of a first copy of a target nucleic acid that has seeded a site, such as delayed or slow activation, compared to the relatively rapid rate at which subsequent copies are made to fill the site. In some embodiments, an individual site may have been seeded with several different target nucleic acids, such as several target nucleic acids can be present at each site prior to amplification. However, first copy formation for any given target nucleic acid can be activated randomly such that the average rate of first copy formation is relatively slow compared to the rate at which subsequent copies are generated. In this case, although an individual site may have been seeded with several different target nucleic acids, kinetic exclusion will allow only one of those target nucleic acids to be amplified. More specifically, once a first target nucleic acid has been activated for amplification, the site will rapidly fill to capacity with its copies, thereby preventing copies of a second target nucleic acid from being made at the site.

An amplification reagent can include further components that facilitate amplicon formation and in some cases increase the rate of amplicon formation. An example is a recombinase. Recombinase can facilitate amplicon formation by allowing repeated invasion/extension. More specifically, recombinase can facilitate invasion of a target nucleic acid by the polymerase and extension of a primer by the polymerase using the target nucleic acid as a template for amplicon formation. This process can be repeated as a chain reaction where amplicons produced from each round of invasion/extension serve as templates in a subsequent round. The process can occur more rapidly than standard PCR since a denaturation cycle, such as via heating or chemical denaturation, is not required. As such, recombinase-facilitated amplification can be carried out isothermally. It is generally desirable to include ATP, or other nucleotides, or in some cases non-hydrolyzable analogs thereof, in a recombinase-facilitated amplification reagent to facilitate amplification. A mixture of recombinase and single stranded binding (SSB) protein is particularly useful as SSB can further facilitate amplification. Example formulations for recombinase-facilitated amplification include those sold commercially as TwistAmp kits by TwistDx (Cambridge, UK). Useful components of recombinase-facilitated amplification reagent and reaction conditions are set forth in US 5,223,414 and US 7,399,590, each of which is incorporated herein by reference in its entirety.

Another example of a component that can be included in an amplification reagent to facilitate amplicon formation and in some cases to increase the rate of amplicon formation is a helicase. Helicase can facilitate amplicon formation by allowing a chain reaction of amplicon formation. The process can occur more rapidly than standard PCR since a denaturation cycle, such as via heating or chemical denaturation, is not required. As such, helicase-facilitated amplification can be carried out isothermally. A mixture of helicase and single stranded binding (SSB) protein is particularly useful as SSB can further facilitate amplification. Exemplary formulations for helicase-facilitated amplification include those sold commercially as IsoAmp kits from Biohelix (Beverly, MA). Further, examples of useful formulations that include a helicase protein are described in US 7,399,590 and US 7,829,284, each of which is incorporated herein by reference in its entirety.

In some embodiments, amplification reagents can include one or more origin binding proteins. Without being bound by any particular theory, the inclusion of an origin binding protein in the amplification reaction facilitates amplicon formation and, in some case increases the rate of amplicon formation.

In some embodiments, the amplification reagents can further include a polymerase. In some embodiments, the polymerase can be a strand-displacing polymerase such as a Bst polymerase, a polD polymerase, a 9°N polymerase or phi29 polymerase. In some embodiments, the polymerase is a thermostable polymerase. In some embodiments, the template is RNA and the polymerase can include a reverse transcriptase.

The maintenance of sample representation or specificity is useful to many library preparation methods for several downstream analytical applications, such as next generation sequencing (NGS) and targeted sequencing. For example, for many cDNA library applications, such as searching for differentially expressed genes, it is useful to minimize the distortion of cDNA representation in a library with respect to initial mRNA population. Stated differently, the content of individual cDNAs in the library, in some downstream applications, can be proportional to the copy number of the initial RNAs. In contrast, for some other applications, the concentrations of different individual cDNAs in a library can be equalized. Embodiments described herein, can maintain the complexity of an input DNA library, including the DNA species-to-species ratio, and possible minor allele frequency calls, with little or no observed difference other than the amount of the library being amplified and normalized.

### Genotyping by sequencing

Some embodiments include methods and systems for genotyping by sequencing a target nucleic acid to identify a SNP, insertion, deletion, or duplication in the target nucleic acid. In some such embodiments, a nucleic acid library comprising a target nucleic acid can be prepared by obtaining a substrate having a plurality of capture probes attached thereto. In some embodiments, a substrate comprises a solid support. In some embodiments, a substrate is a planar substrate. In some embodiments, a planar substrate includes discrete sites, such as wells. In some embodiments, a substrate can include a plurality of beads. In some embodiments, the capture probes comprise first amplification sites and first locus specific primers. Some embodiments include hybridizing a plurality of target nucleic acids to the first locus specific primers. In some embodiments, the first locus specific primers can be extended to obtain extended probes containing second amplification sites. In some embodiments, the extended probes can be amplified and sequenced, thereby identifying a SNP, insertion, deletion, or duplication in the target nucleic acid.

In some embodiments, extending the first locus specific primers can include hybridizing second locus specific primers to the target nucleic acids, and ligating the first locus specific primers to the second locus specific primers. In some embodiments, an extension oligonucleotide can be hybridized to the second locus specific primer, and the second locus specific primer can be extended with a polymerase to include sequences complementary to the extension oligonucleotide. In some embodiments, the sequences complementary to the extension oligonucleotide can include a second amplification site, a second sequencing primer, and/or a second index.

In some embodiments, the extended probes can be amplified to obtain a normalized amount of amplification products. In some embodiments, the amplification is limited by either the amount of the capture probes or the amount of the extension primers. In some embodiments, the substrate comprises a normalizing amount of the capture probes, and the amount of the extension primers is equal to or greater than the normalizing amount of capture probes. In some embodiments, the substrate comprises a normalizing amount of the extension primers, and the amount of the capture probes is equal to or greater than the normalizing amount of capture probes. In some embodiments, the extension primers are attached to the substrate. In some embodiments, the extension primers are in solution.

In some embodiments, the first amplification sites and the extension primers are incapable of or are essentially incapable of hybridizing to one another. In some embodiments, the first amplification sites and the extension primers are non-complementary to one another. In some embodiments, the first amplification sites and the extension primers comprise non-complementary nucleotide sequences to one another. In some embodiments, the first amplification sites comprise modified nucleotides that inhibit hybridization with the extension primers. In some embodiments, the first amplification sites and the extension primers each lack the same type of nucleotide. In some embodiments, the first amplification sites lack types of nucleotides complementary to one another; and the extension primers consist essentially of the same types of nucleotides as the first amplification site. In some embodiments, the first amplification site lacks a combination of nucleotides selected from: adenine (A) and thymine (T); or guanine (G) and cytosine (C). In some embodiments, the first amplification site consists of a combination of nucleotides selected from: adenine (A) and guanine (G); adenine (A) and cytosine (C); cytosine (C) and thymine (T); or guanine (G) and thymine (T). In some embodiments, the first amplification site consists of a combination of adenine (A) and guanine (G) nucleotides.

### Solid supports

Some embodiments include one or more substrates, such as solid supports. Solid supports suitable for the methods disclosed herein can generally be of any convenient size and fabricated from any number of known materials. Preferably, the solid support used in the embodiments disclosed herein can be of any suitable type that provides a known binding capacity, resulting in a substantially unfluctuating amount of bound nucleic acids per fixed amount of solid support. Example of such materials include: inorganics, natural polymers, and synthetic polymers. Specific examples of these materials include: cellulose, cellulose derivatives, acrylic resins, glass, silica gels, gelatin, polystyrene, polyvinyl pyrrolidone, copolymers of vinyl and acrylamide, polystyrene cross-linked with divinylbenzene or the like, polyacrylamides, latex gels, silicon, plastics, nitrocellulose, polystyrene, dextran, rubber, natural sponges, silica gels, control pore glass, metals, cross-linked dextrans (e.g., Sephadex^{™}) agarose gel (Sepharose^{™}), and other solid supports known to those of skill in the art.

In some embodiments, the solid phase supports can include synthetic polymer supports, such as polystyrene, polypropylene, substituted polystyrene (e.g., carboxylated or aminated polystyrene), polyamides, polyacrylamides, polyvinylchloride, and the like, or any material useful in nucleic acid affinity chromatography. In some embodiments, the solid phase can be a flat surface, curved surface, a well, or part of a microfluidic device.

In some embodiments disclosed herein, the solid support can include beads. Beads may be any of a wide variety of shapes, such as spherical, generally spherical, egg shaped, disc shaped, cubical, amorphous and other three dimensional shapes.. Beads may be manufactured using a wide variety of materials, including for example, resins, and polymers. The beads may be any suitable size, including for example, microbeads, microparticles, nanobeads and nanoparticles. In some cases, beads are magnetically responsive; in other cases beads are not significantly magnetically responsive. For magnetically responsive beads, the magnetically responsive material may constitute substantially all of a bead, a portion of a bead, or only one component of a bead. The remainder of the bead may include, among other things, polymeric material, coatings, and moieties which permit attachment of an assay reagent. Examples of suitable beads include flow cytometry microbeads, polystyrene microparticles and nanoparticles, functionalized polystyrene microparticles and nanoparticles, coated polystyrene microparticles and nanoparticles, silica microbeads, fluorescent microspheres and nanospheres, functionalized fluorescent microspheres and nanospheres, coated fluorescent microspheres and nanospheres, color dyed microparticles and nanoparticles, magnetic microparticles and nanoparticles, superparamagnetic microparticles and nanoparticles (e.g., DYNABEADS^{®} particles, available from Invitrogen Group, Carlsbad, CA), fluorescent microparticles and nanoparticles, coated magnetic microparticles and nanoparticles, ferromagnetic microparticles and nanoparticles, coated ferromagnetic microparticles and nanoparticles, and those disclosed in U.S. Patent Pub. No. 20050260686; U.S. Patent Pub. No. 20030132538; U.S. Patent Pub. No. 20050118574; U.S. Patent Pub. No. 20050277197; U.S. Patent Pub. No. 20060159962, the entire disclosures of which are incorporated herein by reference in their entireties. Beads may be pre-coupled with a biomolecule or other substance that is able to bind to and form a complex with a biomolecule. Beads may be pre-coupled with an antibody, protein or antigen, DNA/RNA probe or any other molecule with an affinity for a desired target. Bead characteristics may be employed in embodiments of multiplexing aspects. Examples of beads having characteristics suitable for multiplexing, as well as methods of detecting and analyzing signals emitted from such beads, may be found in U.S. Patent Pub. No. 20080305481; U.S. Patent Pub. No. 20080151240; U.S. Patent Pub. No. 20070207513; U.S. Patent Pub. No. 20070064990; U.S. Patent Pub. No. 20060159962; U.S. Patent Pub. No. 20050277197; U.S. Patent Publication No. 20050118574, the disclosures of which are incorporated herein by reference in their entireties.

In some embodiments, beads can be of any convenient size and fabricated from any number of known materials. In some embodiments, the bead can be, for example, magnetic beads, paramagnetic beads, plastic beads, polystyrene beads, glass beads, agarose beads, and combinations thereof. In some embodiments, the beads are beads approximately 2 to 100 µm in diameter, or 10 to 80 µm in diameter, most preferably 20 to 40 µm in diameter. In some embodiments, the beads can be provided in solution. In some embodiments, or the beads can be immobilized on a solid support.

In some embodiments, the solid support can include streptavidin. In some embodiments, the solid support is, or can include, streptavidin-coated beads. In some embodiments, the solid support is or can include streptavidin-coated magnetic beads. In some embodiments where the solid phase support includes streptavidin, the nucleic acids molecules can be biotinylated to facilitate binding of the nucleic acids to the solid support.

### Input nucleic acids

In some embodiments, an input nucleic acid sample includes single-stranded nucleic acids. In some embodiments, at least one target nucleic acid in an input nucleic acid sample can be double-stranded, or can be rendered at least partly double-stranded using appropriate procedures. In some embodiments, the input nucleic acid sample includes a mixture of single-stranded nucleic acid molecules and double-stranded nucleic acid molecules. In some embodiments, the target nucleic acid molecules can be linear. In some embodiments, the target nucleic acid molecules can be circular, or include a combination of linear and circular regions.

In some embodiments, the amount of an input nucleic acid can be from about 0.01 ng to 100 ng. In some embodiments, the amount of input nucleic acid is about 0.1 ng, 0.2 ng, 0.3 ng, 0.4 ng, 0.5 ng, 0.6 ng, 0.7 ng, 0.8 ng, 0.9 ng, 1 ng, 1.1 ng, 1.2 ng, 1.3 ng, 1.5 ng, 2.0 ng, 2.5 ng, 3.0 ng, 3.5 ng, 4.0 ng, 4.5 ng, 5.0 ng, or within a range defined by any two of the aforementioned values. In some embodiments, the amount of input nucleic acid is about 5.5 ng, 6.0 ng, 6.5 ng, 7.0 ng, 7.5 ng, 8.0 ng, 8.5 ng, 9.0 ng, 9.5 ng, 10.0 ng, 11.0 ng, 11.5 ng, 12.0 ng, 12.5 ng, 13.0 ng, 13.5 ng, 14.0 ng, 15.0 ng, 15.5 ng, 16.0 ng, 16.5 ng, 17.0 ng, 18.0 ng, 18.5 ng, 19.0 ng, 19.5 ng, 20.0 ng, or within a range defined by any two of the aforementioned values. In some embodiments, the amount of input nucleic acid is about 21.0 ng, 22.0 ng, 22.5 ng, 23.0 ng, 23.5 ng, 24.0 ng, 24.5 ng, 25.0 ng, 26.0 ng, 27.0 ng, 28.0 ng, 29.0 ng, 30.0 ng, 32.5 ng, 35.0 ng, 37.5 ng, 40.0 ng, 42.5 ng, 45.0 ng, 47.5 ng, 50.0 ng, 52.5 ng, 55.0 ng, 60.0 ng, 65.0 ng, 70.0 ng, 75.0 ng, 80.0 ng, 85.0 ng, 90.0 ng, or within a range defined by any two of the aforementioned values. In some embodiments, the amount of input nucleic acid is about 0.08, 0.4, 2.0, 10.0, or 50.0 ng.

In some embodiments, an amplification step is performed on a single nucleic acid libraries. In some embodiments, an amplification step is performed on a plurality of nucleic acid libraries. In some embodiments, the amplification products from the plurality of nucleic acid libraries are combined to form a combined pooled nucleic acid library. In some embodiments, the amplification products derived from the plurality of nucleic acid libraries are combined after being removed from the solid phase support. In some embodiments, the amplification products from the plurality of nucleic acid libraries are combined before being removed from the solid phase support. In some embodiments, the plurality of input nucleic acid samples is combined before the amplification step. In some embodiments, the amount of each input nucleic acid sample is not normalized across the plurality of nucleic acid samples.

In some embodiments, the library is generated from DNA. In some embodiments the library is generated from RNA. In some embodiments, the library is generated from proteins. In some embodiments, the library is generated from a combination of DNA, RNA, and protein. For example, antibody-oligo conjugates can be used to generate a library on a solid-support and subsequently normalized through limited amplification on a solid-support.

In some embodiments, the plurality of input nucleic acid samples comprises at least 2, 4, 8, 12, 24, 48, 96, 200, 384, 400, 500, 100, 1500, or a number of input nucleic acid samples within a range defined by any two of the aforementioned numbers.

In some embodiments, the relative representation of each population of constituent amplification products can be advantageously adjusted in the pooled nucleic acid library. By "advantageously adjusted", as used herein, is meant that the amount of each constituent amplification products in the pooled nucleic acid library can be controlled or predetermined. In some embodiments, the amount of each constituent amplification products is substantially uniformly represented in the pooled nucleic acid library. Alternatively, the plurality of constituent amplification products can be present in the pooled nucleic acid library in different, predetermined concentrations. This may be achieved by assembling different amounts of the solid phase supports with amplification products remaining affixed thereon or by pooling different amounts of the plurality of constituent amplification products after being recovered from the solid phase support. Stated differently, the advantageous adjustment can include selectively adjusting both the proportional representation and the population number of constituent amplification products in the pooled nucleic acid library. In some embodiments, an advantageous adjustment may include subjecting a sample of constituent amplification products to at least one processing step in addition to recovering amplification products from the solid phase support.

### Indexing by extension

Some embodiments provided herein include indexing of nucleic acid libraries by extension of indexing oligonucleotides hybridized to common sequences on nucleic acid molecules. Some protocols for DNA library indexing can include multiple enzymatic and purification steps, or can be performed in a final PCR amplification step. Such indexing protocols may also rely on a single round of indexing, thus not supporting combinatorial indexing which can include individually tagging large number of molecules in a single sample with individual combination of indices. Advantageously, embodiments provided herein can include both single and combinatorial indexing, such as multiple levels of indexing. Some embodiments include indexing nucleic acids in solution, or indexing nucleic acids attached to a substrate, such as beads. Some embodiments include indexing nucleic acids, such as DNA, such as crude chromatin bound to beads. Some embodiments include *in situ* indexing of nucleic acids, such as DNA present inside of cell/nuclei.

Some embodiments for preparing an indexed nucleic acid library include adding Y-adaptors to a plurality of target nucleic acids. In some embodiments, a Y-adaptor includes a double-stranded nucleic acid comprising a portion or end in which both strands are hybridized to one another, and a portion or end in which the strands do not hybridize to one another. In some embodiments, the Y-adaptors are added to first and second ends of each target nucleic acid, and each Y-adaptor comprises a first strand comprising a first primer binding site and a mosaic element, and a second strand comprising a second primer binding site and a complement to the mosaic element. Some embodiments also include hybridizing a first extension oligonucleotide to the second primer binding site, wherein the first extension oligonucleotide comprises a first index and a third primer binding site. Some embodiments also include extending the second strand comprising the second primer binding site, thereby adding the first index to the target nucleic acids.

In some embodiments, a 5' end of the first strand of the Y-adaptor is resistant to nuclease degradation. For example, the 5' end of the first strand of the Y-adaptor can include a phosphorothioate bond between at least two consecutive nucleotides. In some embodiments, a 5' end of the second strand of the Y-adaptor is phosphorylated.

In some embodiments, adding Y-adaptors to a plurality of target nucleic acids includes contacting the plurality of target nucleic acids with a plurality of transposomes, wherein each transposome comprises a Y-adaptor and a transposase. In some embodiments, the transposomes comprise dimers. In some embodiments, the transposase comprises a Tn5 transposase. In some embodiments, the transposomes are bound to a substrate. In some embodiments, the substrate comprises a plurality of beads. In some embodiments, the beads are magnetic.

In some embodiments, a 3' end of the first extension oligonucleotide is blocked. In some embodiments, a 5' end of the first extension oligonucleotide is phosphorylated. In some embodiments, the first extension oligonucleotide is bound to a bead. Some embodiments also include cleaving the first extension oligonucleotide from the bead prior to extending the second strand comprising the second primer binding site.

In some embodiments, extending the second strand comprising the second primer binding site comprises polymerase extension, and/or extension with a ligase. In some embodiments, extension with a ligase comprises: hybridizing a ligation oligonucleotide to the first extension oligonucleotide hybridized to the second primer binding site; and ligating the ligation oligonucleotide to the second strand comprising the second primer binding site. In some embodiments, the ligation oligonucleotide comprises an additional index. In some embodiments, the ligation oligonucleotide comprises an additional primer binding site.

Some embodiments also include removing the first extension oligonucleotide after extending the second strand comprising the second primer binding site. In some embodiments, the removing comprises an exonuclease treatment. In some embodiments, the first extension oligonucleotide comprises uracil nucleotides, and the removing comprises degradation of the first extension oligonucleotide with a uracil-specific excision reagent (USER) enzyme.

Some embodiments also include adding a second index to the target nucleic acids comprising the first index. In some embodiments, adding the second index comprises: hybridizing a second extension oligonucleotide to the third primer binding site of the target nucleic acids comprising the first index, wherein the second extension oligonucleotide comprises the second index; and extending the second strand comprising the third primer binding site, thereby adding the second index to the target nucleic acids comprising the first index. In some embodiments, a 3' end of the second extension oligonucleotide is blocked. In some embodiments, a 5' end of the second extension oligonucleotide is phosphorylated.

In some embodiments, extending the second strand comprising the third primer binding site comprises polymerase extension. In some embodiments, extending the second strand comprising the third primer binding site comprises extension with a ligase. Some embodiments also include removing the second extension oligonucleotide after extending the third primer binding site.

Some embodiments also include adding a third index to the target nucleic acids comprising the second index. Some embodiments also include adding an additional index to the target nucleic acids comprising the third index.

Some embodiments also include amplifying the target nucleic acids comprising one or more indexes, such as a first index, second index, third index, or more. In some embodiments, the amplification comprises hybridizing amplification primers to the first primer binding sites and to a third, fourth, or fifth primer binding sites. In some embodiments, the primer binding sites can include generic sequences such as a P5 or P7 sequence or complement thereof. In some embodiments, the amplification comprises a PCR. In some embodiments, the amplification comprises bridge amplification. In some embodiments, the target nucleic acids comprise genomic DNA.

Some embodiments include a method of combinatorial indexing a plurality of target nucleic acids. Some embodiments include (a) obtaining a pool of primary indexed nucleic acids, comprising: adding a first index to a plurality of subpopulations of target nucleic acids, wherein a different first index is added to each subpopulation, and combining the subpopulations comprising the different first indexes to obtain the pool of primary indexed nucleic acids, wherein the first index is added to a subpopulation of target nucleic acids according to any of the foregoing embodiments; (b) splitting the pool into a plurality of subpopulations of primary indexed nucleic acids; and (c) obtaining a pool of secondary indexed nucleic acids, comprising: adding a second index to the plurality of subpopulations of primary indexed nucleic acids, wherein a different second index is added to each subpopulation, and combining the subpopulations comprising the different second indexes to obtain the pool of secondary indexed nucleic acids. Some embodiments also include repeating (b) and (c) and adding additional indexes to indexed subpopulations. In some embodiments, adding a first index to a subpopulation of target nucleic acids is performed in a compartment selected from a well, a channel, or a droplet.

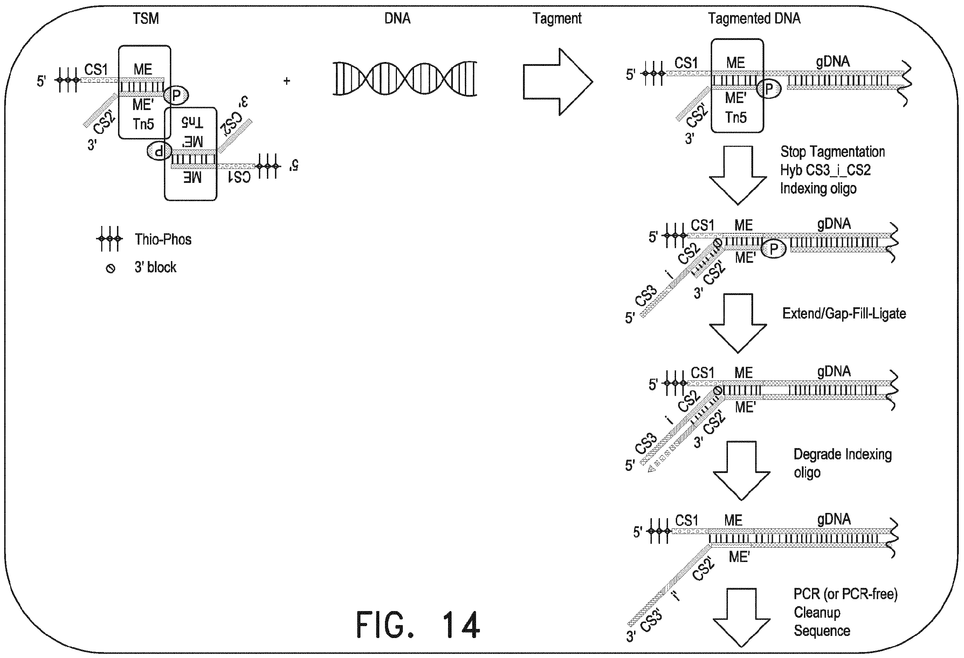

An example of a single level indexing by extension of tagmented DNA in solution is presented in FIG. 14. Briefly, FIG. 14 shows single level indexing by hybridization-extension in solution. DNA is tagmented in solution by Y-TSM, followed by TSM inactivation, indexing oligo annealing and extension. If needed exonuclease treatment and final PCR amplification is performed. Thus, in a first step, genomic DNA is tagmented with a transposome (TSM), such that the transposome inserts into the DNA and fragments the DNA. The transposome includes nucleic acids containing mosaic end (ME) sequences, and common sequences CS1 and CS2'. Insertion of the transposome sequences into the DNA results in the DNA have an end with a Y-adaptor. Each DNA fragment can be flanked by different common sequences on either end. In some embodiments, several nucleosides at the 5' end of the transposome nucleic acids, such as the transfer strand, can include phosphorothioate bonds resistant to nuclease degradation. As shown in FIG. 14, tagmentation can be stopped, and transposomes can be removed, such as by treatment with SDS. An indexing oligo, such as CS3_i1_CS2 indexing oligo, can be hybridized to the Y-adaptor. A mix of DNA polymerase and ligase can be used to fill up and ligate 9 base gap generated during tagmentation on the non-transfer strand, and to extend over hybridized indexing oligo. A cocktail or a single 5' exonuclease can be added to remove excess indexing oligonucleotides. PCR can be performed with sequencing adapter primers containing CS1 and CS3 sequences. However, exonuclease treatment can be skipped if relatively low concentrations of indexing oligo were used, or indexing oligonucleotides were first hybridized to tagmented DNA still bound by Tn5. DNA can be purified using a column prior to extension reaction. In some embodiments, indexing primers can be synthesized with uracil bases instead of thymine, and then removed by degradation with an enzyme, such as uracil-specific excision reagent (USER) enzyme. In some embodiments that include a PCR-free workflow, CS1 and CS3 can contain P5 and P7 sequences which can be utilized for clustering on a flow cell.

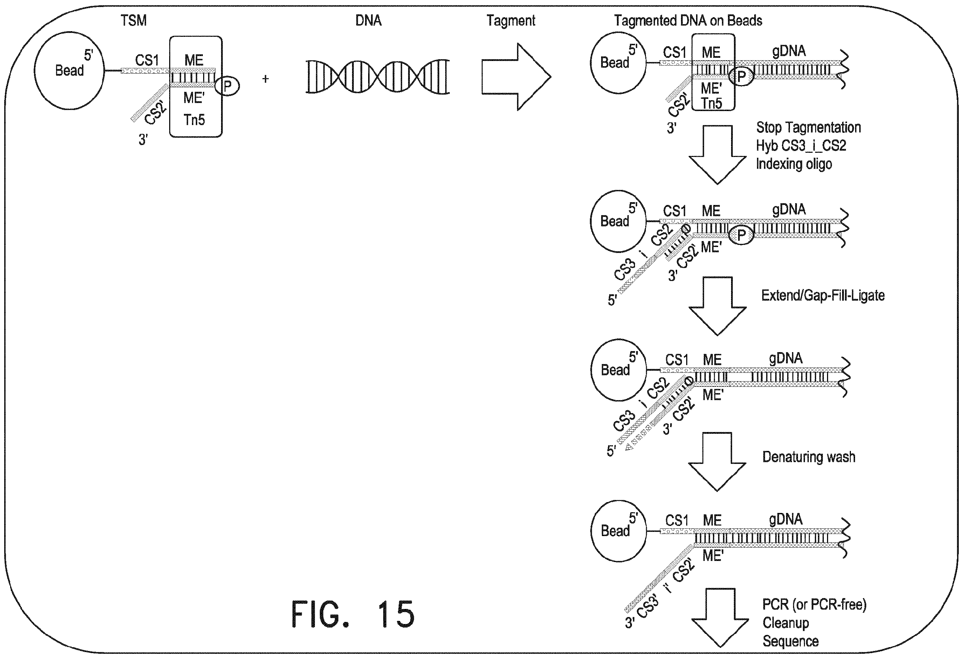

Some embodiments of an indexing workflow applied for DNA tagmented on beads is shown in FIG. 15. Briefly, FIG. 15 shows an embodiment of single level indexing by hybridization-extension on beads in which DNA is tagmented by transposomes with Y adapters prebound to magnetic beads, and all consequent steps including TSM inactivation, indexing oligo annealing and gap-fill/ligation/extension are done by resuspension and pelleting the beads in different buffers. An advantage of performing indexing by extension can be observed in combinatorial indexing workflows, such as the addition of multiple indices in a step wise manner in a series of split and pool reactions. Depending on numbers of indexing levels combinatorial indexing can include individual indexing of millions of molecules, such as from a single cell, with a relatively small pool, such as hundreds, of initial indices.

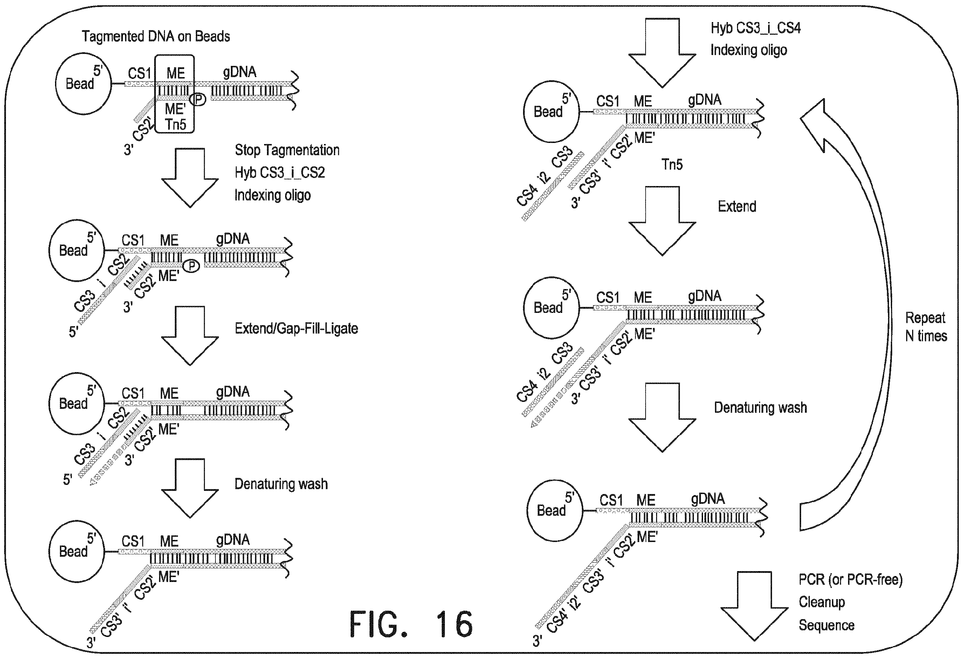

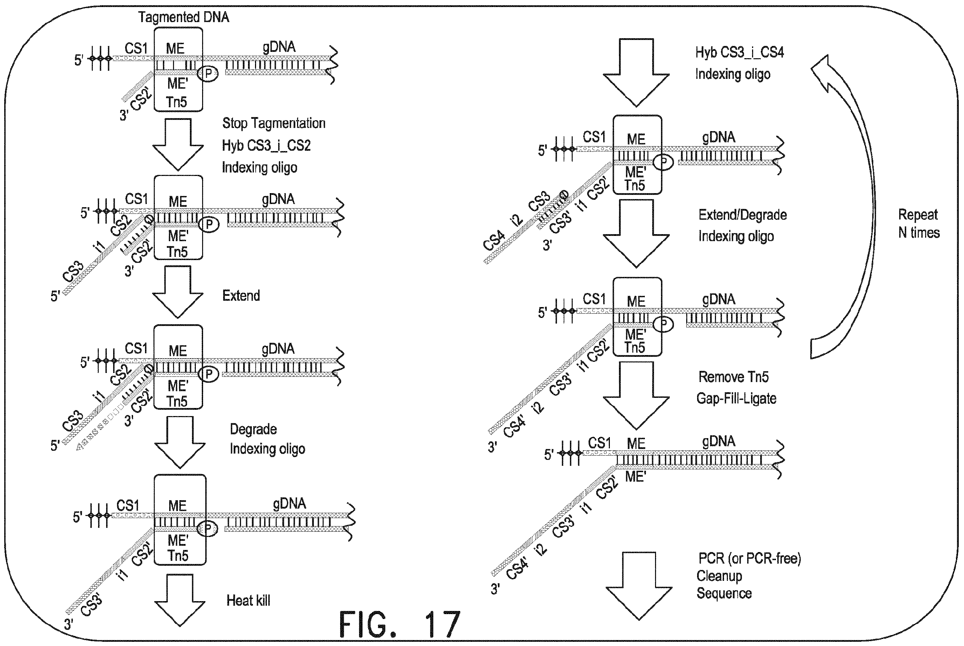

Some embodiments that include the addition of multiple indices to a DNA molecule are depicted in FIG. 16 and FIG. 17. FIG. 16 depicts an example embodiment of addition of indices by extension to DNA tagmented on beads. In some embodiments, Tn5 is removed during a stop tagmentation step and a non-transfer strand is gap-fill-ligated simultaneously with the first level indexing. The removal of indexing oligonucleotides is achieved by bead wash with NaOH, followed by neutralization and next level indexing primer hybridization. FIG. 17 depicts an embodiment that includes tagmentation in solution. In some embodiments, indexing extension steps are done with Tn5 being bound to DNA filament in order to keep DNA contiguity. Tn5 removal and non-transfer strand gap-fill-ligation is performed at the last step of the workflow, right before PCR. Indexing primers are released either by 5' specific exonuclease treatment, or indexing primers are synthesized with uracil instead of thymine, and post extension complexes are treated with USER enzyme. Not shown in FIG. 16 and FIG. 17 are splitting-pooling cycles included in embodiments of a combinatorial indexing workflow. In the embodiments depicted in FIG 16 and FIG. 17, after the addition of the first index, indexing oligonucleotides can be washed away, such as in a bead based workflow of FIG. 16, or degraded, such as in a solution workflow of FIG. 17. A second level of indexing can proceed in which second level oligonucleotides can be hybridized, followed by its extension and removal. In some embodiments, cycles of indexing primer hybridization/extension/removal can be repeated multiple times depending on required plexity of the assay.

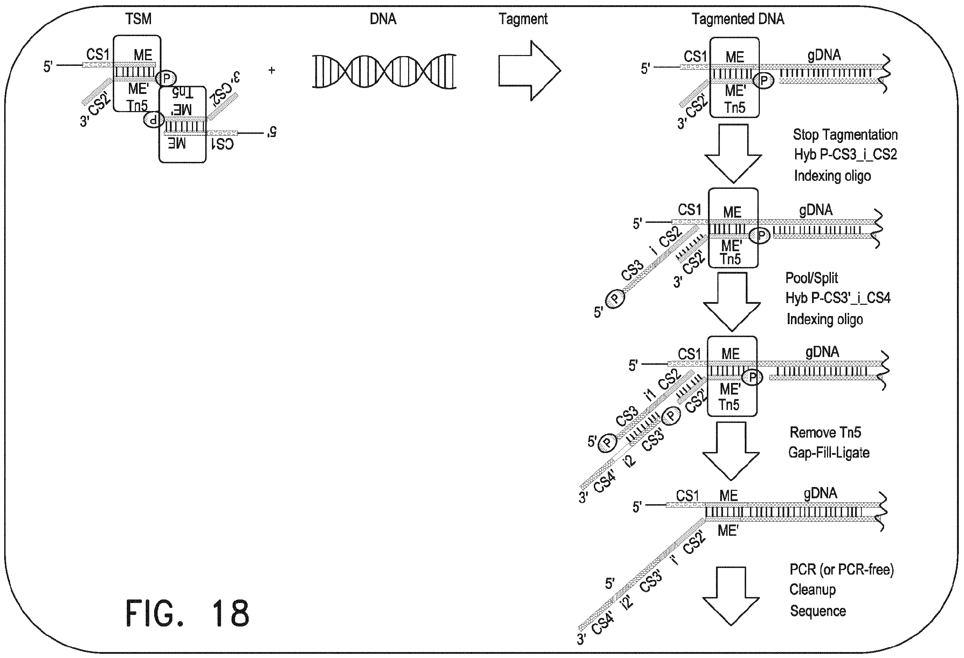

In some embodiments, nucleic acids may degrade with increasing number of cycles. To reduce degradation of nucleic acids, such as oligonucleotides, some embodiments can include one or more method steps depicted in FIG. 18. In some embodiments, a three level indexing can be performed using multiple hybridizations-single extension/ligation steps in solution. For example, in some embodiments, tagmented cells with bound Tn5 are distributed in wells of microtiter plate. The contents of each well can be hybridized to individual first level 5' phosphorylated indexing oligonucleotides. Excess unhybridized indexing oligonucleotides can be removed. Cells can be pooled and split into well of a microtiter plate with a set second 5' phosphorylated indexing oligonucleotides. Second indexing oligonucleotides can be annealed to the sticky 5' ends of the first indexing oligonucleotides, followed by Tn5 removal and gap-fill-ligation of both gaps on non-transfer strand. Library elements can be pooled and either used in a PCR-free two level indexing workflow, or split for PCR amplification with indexing primers, to add the third level of indexing. By using 5' phosphorylated indexing oligonucleotides at each indexing step this protocol can be extended from three to multiple level indexing. This workflow can be used with single cell ATAC-seq, since the excess of indexing oligonucleotides can be washed away at cell pooling steps. Some embodiments include the use of similar protocols with DNA tagmented on beads for long linked reads applications.

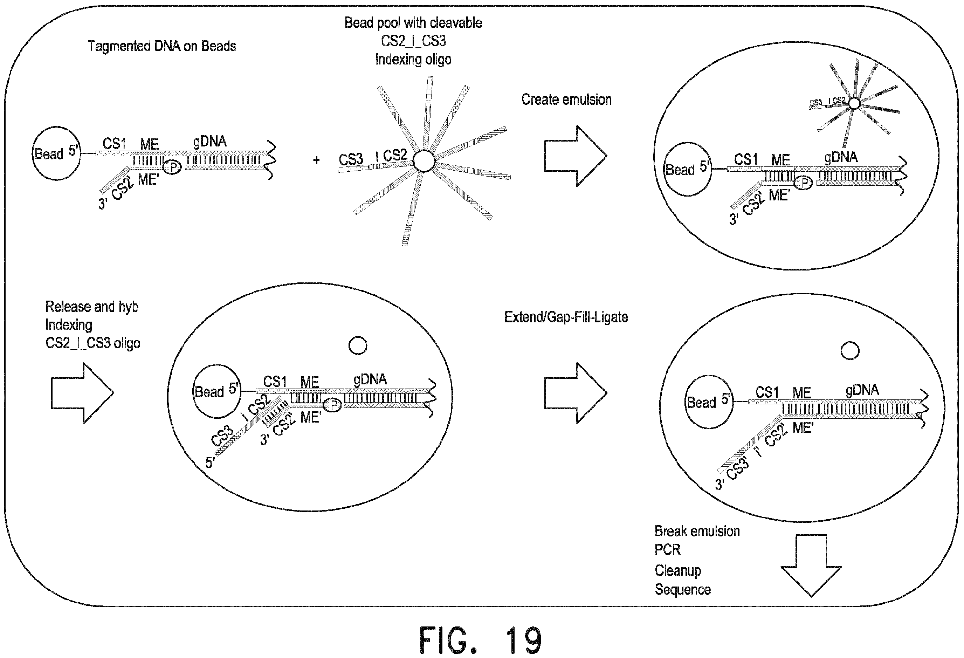

In some embodiments, indexing by hybridization-extension can be performed in individual compartments containing a single indexing bead from a large pool of beads covered by unique cleavable indexing oligonucleotides. Examples of compartments include wells in a microwell plate, Fluidigm-like channels, or droplets. To provide phasing information or single cell resolution, tagmented DNA for indexing can be either prebound to beads, or be contained inside of nuclear envelope. An example embodiment of single level indexing of bead bound tagmented DNA by hybridization-extension in droplets is depicted in FIG. 19. Single level indexing by hybridization-extension in droplets can include DNA tagmented with Y adapter transposomes prebound to beads which are emulsified with a pool of beads/hydrogel particles individually covered by cleavable indexing oligonucleotides and other components required for oligonucleotide cleavage and extension. Droplets can be generated either by droplet generator or with emulsion reagents aiming to encapsulate one tagmentation and one indexing bead per vessel. Upon encapsulation indexing oligonucleotides are released, hybridized to tagmented DNA and extended. Indexed DNA is PCR amplified after emulsion is broken.

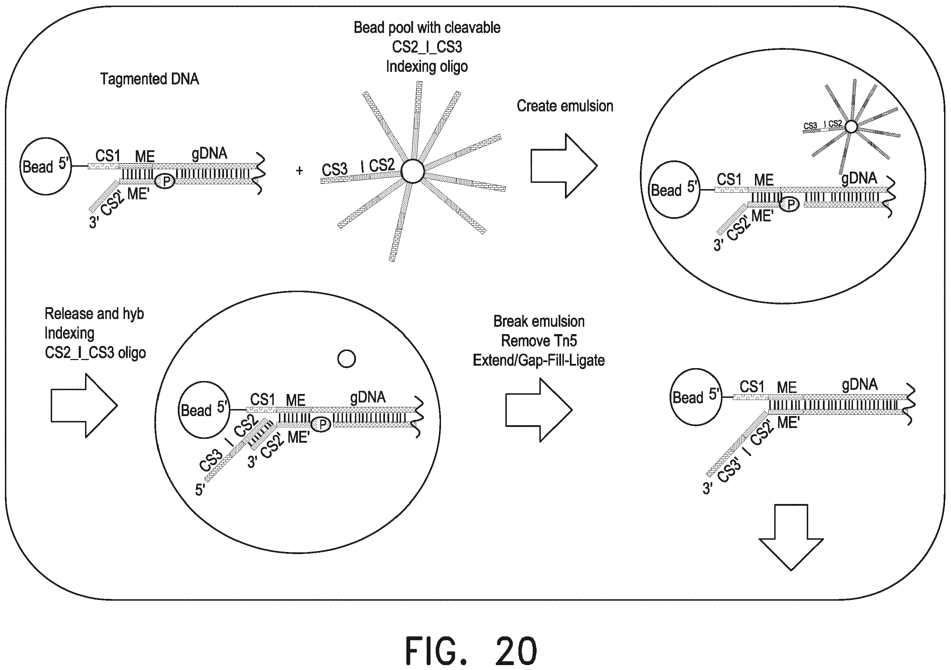

In some embodiments, droplets can be used for a hybridization step. In some embodiments, subsequent extension steps can be performed in bulk after contents of the droplets is released. FIG. 20 depicts an embodiment that includes single level indexing by hybridization in droplets-in bulk extension. In some embodiments, tagmented DNA is emulsified with a pool of beads/hydrogel particles individually covered by cleavable indexing oligonucleotides, and only components for oligo cleavage are included. Upon encapsulation, indexing oligonucleotides are released and hybridized to tagmented DNA. The following steps including extension of indexing oligonucleotides can be performed after the emulsion is broken.

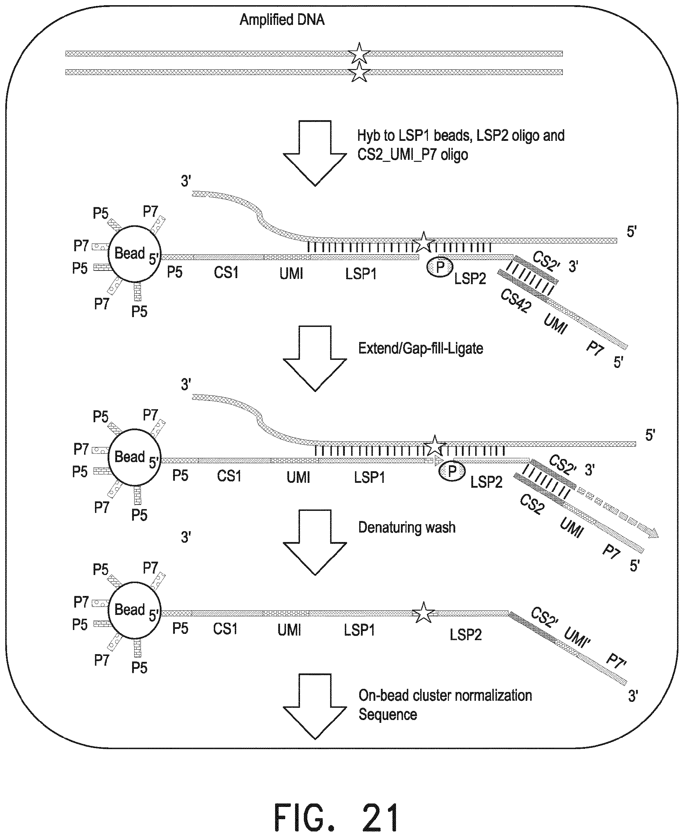

Some embodiments also include DNA molecules enriched on beads grafted by oligonucleotides containing locus specific sequences. Briefly, a sample indexing oligonucleotide is added to the enrichment/hybridization mixture and extended at the same time as the gap between locus specific enrichment oligonucleotides is sealed in post enrichment step. As shown in FIG. 21, amplified genomic DNA is denatured and hybridized to a locus specific probe (LSP1) on a capture bead, designed immediately upstream from the SNP of interest. Simultaneously, 5' phosphorylated LSP2 oligonucleotides containing CS2' sequence are hybridized one or a few bases downstream from the same SNP. The hybridization mixture is also supplemented with an indexing oligonucleotide, containing an index sequence flanked by CS2 and P7 common sequences. Unbound library, excess LSP2, and indexing oligonucleotides are washed by stringent washes, followed by LSP1 and LSP2 extension over the SNP and indexing oligonucleotides, respectively. Extended and ligated construct is washed under denaturing conditions to remove hybridized genomic DNA and indexing oligo prior to normalization/amplification reaction.

### Increasing efficiency of polymerization reactions

Nucleic acid amplification reactions include the use of polymerases which extend primers by incorporating deoxynucleoside triphosphates (dNTPs) at the 3' ends of primers in the presence of catalytic metals, such as Mg²⁺. A forward polymerization reaction includes addition of a nucleotide to a primer to generate an extended primer and inorganic pyrophosphate (PPi); however, the reverse reaction is possible, and increasing amounts of PPi can inhibit polymerization.

PPi accumulation is exacerbated when reaction conditions yield large quantities of DNA, such as > 0.4 ug/uL during isothermal whole-genome amplification reactions. The accumulation of a magnesium PPi complex can be visualized as a white visible precipitate. The degree of observed precipitate is time dependent and increases with increasing time in whole genome amplification reactions. However, precipitation in amplification reactions may interfere with applications where high DNA yield is desired, such as single-cell genomics applications. DNA extension in nanowells may be impacted because the local concentration may be elevated due to the confined volumes, such as DNA clusters in nanowells as utilized during SBS chemistry events.

Some embodiments of the methods and compositions provided herein include modifying a nucleic acid comprising: amplifying or extending the nucleic acid in the presence of a polymerase. In some embodiments, the amplifying and/or the extending is performed under conditions suitable to remove PPi, to inhibit pyrohosphorolysis, and/or inhibit formation of a Mg²⁺-PPi complex. In some embodiments, the PPi is soluble. In some embodiments, the amplifying and/or the extending is performed in the presence of an inorganic pyrophosphatase. In some embodiments, a rate of achieving a yield of a product of the amplifying and/or the extending is increased in the presence of the inorganic pyrophosphatase compared to a rate of achieving a yield of a product of the amplifying and/or the extending in the absence of the inorganic pyrophosphatase. In some embodiments, the rate of achieving a yield of a product of the amplifying and/or the extending is increased by at least 2-fold, at least 3-fold, or at least 5-fold. In some embodiments, the amplifying and/or the extending is performed under isothermal conditions. In some embodiments, the amplifying and/or the extending comprises performing a reaction selected from a PCR, a bridge amplification, a whole genome amplification, a loop-mediated isothermal amplification (LAMP), an amplification from nucleic acids obtained from a single cell, a sequencing by synthesis (SBS) reaction, and an exclusion amplification (ExAMp).

In some embodiments, the amplifying and/or the extending comprises performing an amplification from nucleic acids obtained from a single cell, or from cell-free nucleic acids. In some embodiments, the amplifying and/or the extending comprises performing a sequencing by synthesis (SBS) reaction. In some embodiments, the amplifying and/or the extending comprises performing a bridge amplification.

In SBS, extension of a nucleic acid primer along a nucleic acid template (e.g. a target nucleic acid or amplicon thereof) is monitored to determine the sequence of nucleotides in the template. The underlying chemical process can be polymerization (e.g. as catalyzed by a polymerase enzyme). In a particular polymerase-based SBS embodiment, fluorescently labeled nucleotides are added to a primer (thereby extending the primer) in a template dependent fashion such that detection of the order and type of nucleotides added to the primer can be used to determine the sequence of the template. In some embodiments, SBS includes pyrosequencing. Pyrosequencing detects the release of PPi as particular nucleotides are incorporated into a nascent nucleic acid strand (Ronaghi, et al., Analytical Biochemistry 242(1), 84-9 (1996); Ronaghi, Genome Res. 11(1), 3-11 (2001); Ronaghi et al. Science 281(5375), 363 (1998); U.S. Pat. Nos. 6,210,891; 6,258,568 and 6,274,320, each of which is incorporated herein by reference in its entirety). In pyrosequencing, released PPi can be detected by being converted to adenosine triphosphate (ATP) by ATP sulfurylase, and the level of ATP generated can be detected via luciferase produced photons. Thus, the sequencing reaction can be monitored via a luminescence detection system. Flow cells provide a convenient format for housing amplified nucleic acid molecules produced by some methods and compositions provided herein. One or more amplified nucleic acid molecules in such a format can be subjected to an SBS or other detection technique that involves repeated delivery of reagents in cycles. For example, to initiate a first SBS cycle, one or more labeled nucleotides, DNA polymerase, etc., can be flowed into/through a flow cell that houses one or more amplified nucleic acid molecules. Those sites where primer extension causes a labeled nucleotide to be incorporated can be detected. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for embodiments that use reversible termination, a deblocking reagent can be delivered to the flow cell (before or after detection occurs). Washes can be carried out between the various delivery steps. The cycle can then be repeated n times to extend the primer by n nucleotides, thereby detecting a sequence of length n. Example SBS procedures, fluidic systems and detection platforms that can be readily adapted for use with methods and compositions provided herein are described, for example, in Bentley et al., Nature 456:53-59 (2008), WO 04/018497; U.S. Pat. No. 7,057,026; U.S. Pat. No. 7,329,492; U.S. Pat. No. 7,211,414; U.S. Pat. No. 7,315,019; U.S. Pat. No. 7,405,281, each of which is incorporated herein by reference.

Some embodiments include amplification and/or extension of nucleic acids comprising oligonucleotide extension and ligation, rolling circle amplification (RCA) (Lizardi et al., Nat. Genet. 19:225-232 (1998), which is incorporated herein by reference in its entirety) and oligonucleotide ligation assay (OLA). *See e.g.,* U.S. Pat. Nos. 7,582,420, 5,185,243, 5,679,524 and 5,573,907; EP 0320308; EP 0336731; EP 0439182; WO 90101069; WO 89/12696; and WO 89109835, each of which is incorporated herein by reference in its entirety. It will be appreciated that these amplification methodologies can be designed to amplify immobilized nucleic acid fragments. For example, in some embodiments, the amplification method can include ligation probe amplification or oligonucleotide ligation assay (OLA) reactions that contain primers directed specifically to the nucleic acid of interest. In some embodiments, the amplification method can include a primer extension-ligation reaction that contains primers directed specifically to the nucleic acid of interest. As a non-limiting example of primer extension and ligation primers that can be specifically designed to amplify a nucleic acid of interest, the amplification can include primers used for the GoldenGate assay (Illumina, Inc., San Diego, Calif.) as exemplified by U.S. Pat. Nos. 7,582,420 and 7,611,869, each of which is incorporated herein by reference in its entirety.

Example isothermal amplification methods include Multiple Displacement Amplification (MDA) as exemplified by, for example, Dean et al., Proc. Natl. Acad. Sci. USA 99:5261-66 (2002) or isothermal strand displacement nucleic acid amplification as exemplified by, for example U.S. Pat. No. 6,214,587, each of which is incorporated herein by reference in its entirety. Other non-PCR-based methods that can be used in the present disclosure include, for example, strand displacement amplification (SDA) which is described in, for example Walker et al., Molecular Methods for Virus Detection, Academic Press, Inc., 1995; U.S. Pat. Nos. 5,455,166, and 5,130,238, and Walker et al., Nucl. Acids Res. 20:1691-96 (1992) or hyperbranched strand displacement amplification which is described in, for example Lage et al., Genome Research 13:294-307 (2003), each of which is incorporated herein by reference in its entirety.

Some embodiments include amplification such as bridge amplification. Examples of bridge amplification are disclosed in U.S. 20200181686, U.S. 20190374923, U.S. 20180291444 and U.S. 20170342406 which are each incorporated by reference in its entirety. In some embodiments, bridge amplification can include double stranded or partially double stranded amplicons immobilized on a solid support and produced from an extension reaction, which are denatured and the immobilized strand annealed to a universal capture primer through hybridization to an adapter. The resulting structure is immobilized at both ends to create a bridge and the universal capture primer can be extended and then amplified using, for example, a universal primer region contained in the target specific capture primer. This second universal capture primer can be different than the first universal primer complementary to the adapter sequence. The strand that is not immobilized can, for example, be washed away. Bridge amplification can result in a uniform cluster or colony number for amplicons within a plurality. In some embodiments, amplification can include exclusion amplification. *See e.g.,* U.S. 20200181686 and U.S. 20190374923.

### Systems and Kits

Some embodiments relate to systems and kits comprising a substrate having capture probes attached to the substrate. In some embodiments, the substrate comprises a plurality of beads. Some embodiments also include extension primers In some embodiments, the extension primers are in solution. In some embodiments, the capture probes comprises first amplification sites. In some such embodiments, the first amplification sites and the extension primers are incapable of or are essentially incapable of hybridizing to one another. In some embodiments, the first amplification sites and the extension primers are non-complementary to one another. In some embodiments, the first amplification sites and the extension primers comprise non-complementary nucleotide sequences to one another. In some embodiments, the first amplification sites comprise modified nucleotides that inhibit hybridization with the extension primers. In some embodiments, the first amplification sites and the extension primers each lack the same type of nucleotide. In some embodiments, the first amplification sites lack types of nucleotides complementary to one another, and each extension primer consists essentially of the same types of nucleotides as the first amplification site. In some embodiments, the first amplification sites and extension primers lack types of nucleotides complementary to one another, and each extension primer. For example, the first amplification sites lack a combination of nucleotides selected from: adenine (A) and thymine (T); or guanine (G) and cytosine (C). In some embodiments, the first amplification sites consist of a combination of nucleotides selected from: adenine (A) and guanine (G); adenine (A) and cytosine (C); cytosine (C) and thymine (T); or guanine (G) and thymine (T). In some embodiments, the first amplification sites consist of a combination of adenine (A) and guanine (G) nucleotides. In some embodiments, the first amplification site can consist of one type of nucleotide. In some embodiments, the system or kit includes an extension primer. In some embodiments, the extension primer consists of the same types of nucleotides as the capture probes. In some embodiments, the extension primers are provided in an amount less than or equal to the amount of the capture probes. In some embodiments, a system or kit can also include a transposome, a ligase, a polymerase, and/or an inorganic pyrophosphatase.

### EXAMPLES

### Example 1-Genotyping by sequencing with GGA/AAG primers

A method was developed which utilized a "GGA/AAG" primer system. A GGA primer had the sequence of SEQ ID NO:03 (AAAAAGGAGGAGGAGGAGGAGGAAAA); a AAG primer had the sequence of SEQ ID NO:04 (AAGAAGAAGAAGAAGAAGAAGAAGAAGAAA). In this method, both an on-bead primer (capture probe) and an in-solution primer (extension primer) did not include T or C nucleotides, and were used to drive isothermal amplification with Ex-amp. The primers could not interact with themselves or each other, and as a result would not form oligo dimers on the surface of the bead.

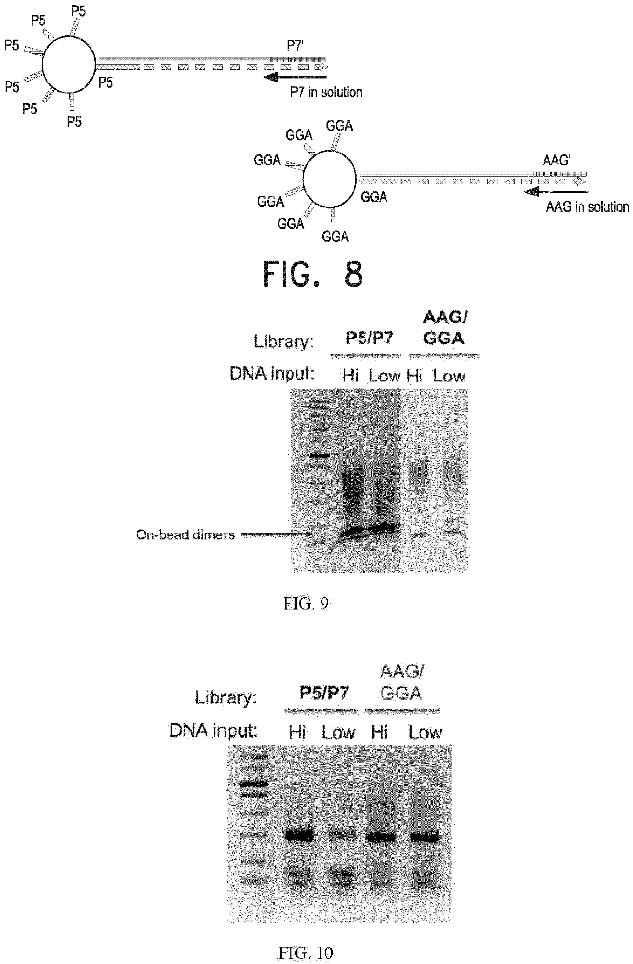

To compare the P5/P7 and GGA/AAG primer systems, two bead types were generated by grafting on the surface either GGA or P5 primers (FIG. 8). A PhiX library with P5/P7 adapters was hybridized to the P5 beads, and a PhiX library with AAG/GGA adapters was hybridized to the GGA beads. Different amounts of the libraries, and a low amount and a high amount of the libraries were hybridized to the beads. The beads were washed and resuspended with ExAMP reagents and supplemented with either P7 primers in solution or AAG in solution primers. Isothermal amplification was performed in a hybridization oven rotating for 1 hour at 38°C. Post-amplification, the beads were washed and further amplified with a PCR with corresponding sets of common primers, targeting a 200 bp region of PhiX genome, and amplified material was analyzed on an agarose gel (FIG. 9 and FIG. 10). The foregoing workflows were substantially similar to the workflows depicted in FIG. 5 and FIG. 6. Significant primer dimerization product was observed for the P5/P7 library, and primer dimerization was significantly reduced with the GGA/AAG primer system (FIG. 9). Gel analysis of amplified material showed significantly more equal yield for libraries generated with the AAG/GGA adapters (FIG. 10). The GGA/AAG primer system yielded more normalized amounts of output DNA for different amounts of input DNA, than the P5/P7 system.

### Example 2-Efficiency of GGA/AAG on-bead normalization

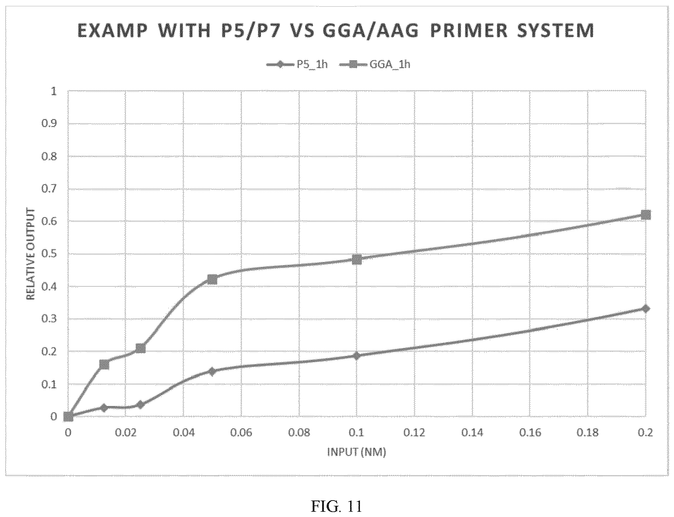

In a substantially similar study to Example 1, the relative amounts of output DNA was measured for various amounts of input DNA using either the P5/P7 primer system, or the GGA/AAG primer system. TABLE 1 and FIG. 11 summarize the results. TABLE 1 shows that the relative amounts of output DNA using the GGA/AAG primer system were substantially similar for different amounts of input DNA.

**TABLE 1**

| Primer system | P5/P7 | GGA/AAG |
|---|---|---|
| Input ratio (high:low) | 8X | 8X |
| Output ratio (high:low) | 9.1X | 2.9X |

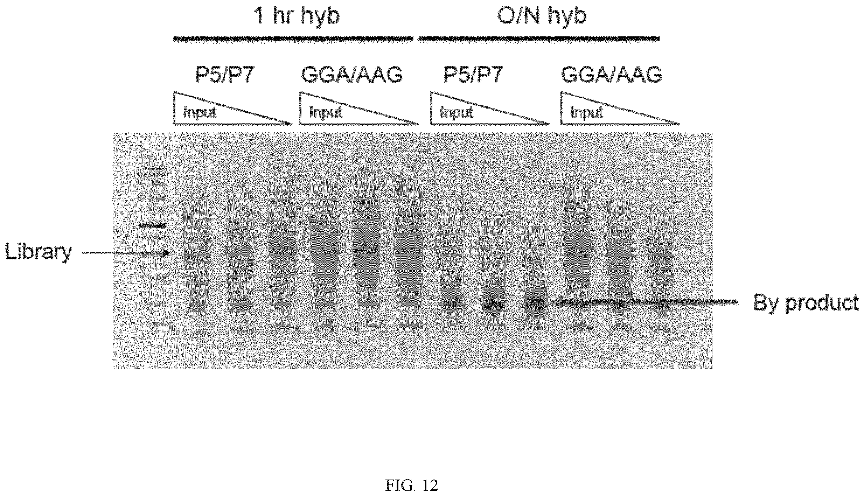

Libraries were hybridized to beads for either 1 hour, or overnight, for various amounts of the library input DNA. FIG. 12 shows that overnight hybridization using the P5/P7 system resulted in a significant amount of by-products.

### Example 3-Genotyping by sequencing with GGA/AAG or P5/P7 primer systems

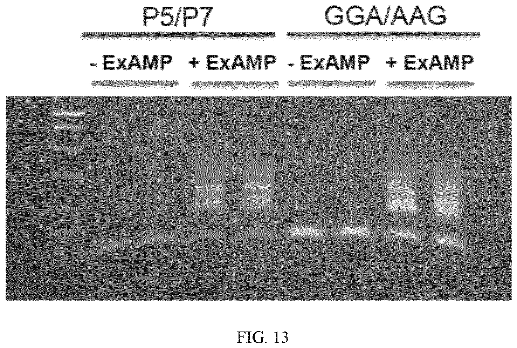

The GGA/AAG or P5/P7 primer systems were compared in a study with 12-plex bead pools. Briefly, 4.8 M beads/condition (800 K each bead type); replicates for each bead pool were used. Human gDNA was whole genome amplified, and about 50 µg of the amplified DNA was hybridized overnight in 24% formamide at room temperature with capture probes on the beads. Non-hybridized DNA was washed from the beads, capture probes were extended, hybridized DNA removed from the extended capture probes, and the capture probes amplified using either P7 or AAG primers in solution. Amplified products were analyzed by quantitative PCR (qPCR). FIG. 13 shows example qPCR products with and without on-bead amplification. Higher amplification yields were observed using the GGA/AAG primer system.

### Example 4-Genotyping by sequencing with GGA/AAG primer system

An example protocol substantially similar to the protocol used in Example 3 is provided. The nomenclature of the primers is consistent with FIG. 5 and FIG. 6. Steps included an overnight hybridization; a gap-fill ligation; exAMP normalization; single-step exAMP and/or two-step exAMP; and sequencing.

Over-night Hybridization: (1) Annealed LSP2 and index primer in a 10:9 ratio (LSP2:index ratio corresponded to 5 µM : 4.5 µM) in 1X annealing buffer (10 mM Tris-HCl, pH 7.5, 100 mM NaCl, 0.1 mM EDTA) and kept on ice. (2) Prepared LSP1 bead pool by mixing equal amounts of a panel of beads individually pre-bound with capture oligos for specific genomic loci, and "GGA-amplification" oligo (SEQ ID NO:05) that served in later steps as a primer for ExAMP normalization. (3) Added to each sample tube desired amount of LSP1 bead pool (example: 500 LSP1 beads/locus) and brought the total number of beads in each tube to 5 million with empty PMP beads. The empty PMP beads were used to decrease bead loss. (4) In a separate tube mixed 5 µl previously prepared whole genom amplified (WGA) DNA, 17 µl of Illumina RA1 buffer, and heat denature at 95°C for 5 min. (5) During WGA DNA denaturation magnetized the beads, removed the supernatant, and transferred 22 µl of the denatured WGA DNA in RA1 buffer to beads pellets and resuspended. (6) Spiked in 2 µl of the 5:4.5 µM LSP2:index duplex. The LSP2:index duplex was at ~400:360 nM final. (7) Performed over-night hybridization at 48°C with constant rotation or frequent agitation.

Gap-fill ligate: (1) After incubation, magnetized the beads and discarded the supernatant. (2) Resuspended the beads in 100 µl of prewarmed to 42°C RA1 buffer. Incubated beads at 42°C for 5 min. (3) Repeated heated wash step 2 more times. (4) Washed 1X with 100 µl GBS-wash buffer (100 mM Tris-HCl, pH 7.5, 100 mM NaCl, 0.1% Tween20). (5) Magnetized the beads, removed supernatant, and resuspended in 20 µl Illumina ELM3 mix. Incubated beads at room temperature for 30 min. (6) After incubation, magnetized the beads and discarded the supernatant. (7) Washed 2X with 100 µl GBS-wash buffer. (8) Magnetized the beads, discarded the supernatant, and resuspended beads in 20 µl 0.1N NaOH. Incubated beads for 5 min at room temperature. (9) Magnetized the beads, removed supernatant, and washed 3X with 100 µl GBS-wash buffer.

ExAMP normalization: (1) Prepared ExAMP reagents as follows (for 8 samples): Mixed 140 µl EPX1, 20 µl EPX2, 74 µl EPX3, 200 nM final in-solution 'AAGGGGT' primer (SEQ ID NO:06). (2) Magnetized the beads, removed supernatant, resuspended beads in 5 µl RSB, and added 20 µl EPX/in-solution primer mix to the beads. Proceeded with Single-step or Double-step ExAMP protocol.

Single-step ExAMP: (1) Incubated the beads at 38°C for 15 min. After 15 min, spiked in 350 nM final 'P5-GGA' (SEQ ID NO:07) and 350 nM final 'AAGGGGT-P7' (SEQ ID NO:08) adapter primers. (2) Incubated the beads for an additional 45 min at 38°C rotating.

Two-step ExAMP: (1) Incubated the beads at 38°C for 1 hr. (2) After incubation, prepared new ExAMP reagents as follows (for 8 samples): Mixed 140µl EPX1, 20 µl EPX2, 74 µl EPX3, 500 nM 'P5-GGA' primer (SEQ ID NO:07), and 500 nM 'AAGGGGT-P7' (SEQ ID NO:08) primer. (3) Magnetized beads, removed supernatant, and washed 1X with GBS wash buffer. (4) Resuspended beads with 5 µl RSB and 20 µl new ExAMP/primer mix. (5) Incubated the beads for an additional for 1 hr at 38°C rotating.

Sequencing: (1) After incubation, samples were cleaned up with 5 µg Zymo column, quantified with Qubit, and clustered for sequencing. (2) Sequencing was performed with a single read and single index run as follows: Read 1: 76 bps with 'P5-GGA' Read 1 primer (SEQ ID NO:07). Index 1: 8 bps with CS' Index 1 primer (SEQ ID NO:09). TABLE 2 lists sequences of certain primers.

**TABLE 2**

| **SEQ ID NO** | **Primer** | **Sequence** |
|---|---|---|
| SEQ ID NO:05 | GGA-amplification | AAAAAGGAGGAGGAGGAGGAGGAAAA |
| SEQ ID NO:06 | 'AAGGGGT' primer | GGGAAGAAGTAAGAAGAATGAAGTAAGAAG ATAGAAA |
| SEQ ID NO:07 | P5-GGA | AATGATACGGCGACCACCGAGATCTACACGG AGGAGGAGGAGGAGGAAAA |
| SEQ ID NO:08 | AAGGGGT-P7 | CAAGCAGAAGACGGCATACGAGATGGGAAG AAGTAAGAAGAATGAAGTAAGAAGATAGAA A |
| SEQ ID NO:09 | CS' Index 1 primer | CTCCACACTACCTCAACCATCACT |

### Example 5-Accelerated and efficient nucleic acid amplification

In this example, a whole genome amplification reaction was performed to determine if PPi accumulation had an adverse effect on reaction kinetics and inhibited polymerization. DNA yield during the amplification was measured with a PicoGreen dsDNA Quantification kit (Molecular Probes). DNA yield reached 80 µg after 3 hours at 37°C, and remained substantially constant up to 24 hours. Increasing the amount of the dNTPs did not increase the rate of the reaction.

An insoluble precipitate accumulated in reaction tubes during DNA amplification. It was hypothesized that the insoluble material was an PPi precipitate. The precipitate was treated with 0.1 to 1 unit inorganic pyrophosphatase (iPPase) (New England Biolabs, M0361L) post DNA amplification. However, attempts to hydrolyze the precipitate were unsuccessful. The iPPase activity may have been suppressed by insoluble PPi substrate and/or the iPPase might lack activity with a magnesium pyrophosphate complex as compared to an uncomplexed PPi substrate. It was hypothesized that the accumulation of a magnesium PPi complex could inhibit the reaction kinetics via the following two mechanisms: (1) PPi ions driving the reaction in the pyrophosphorolysis direction; and (2) Mg²⁺ ion depletion due to the formation of the magnesium pyrophosphate species.

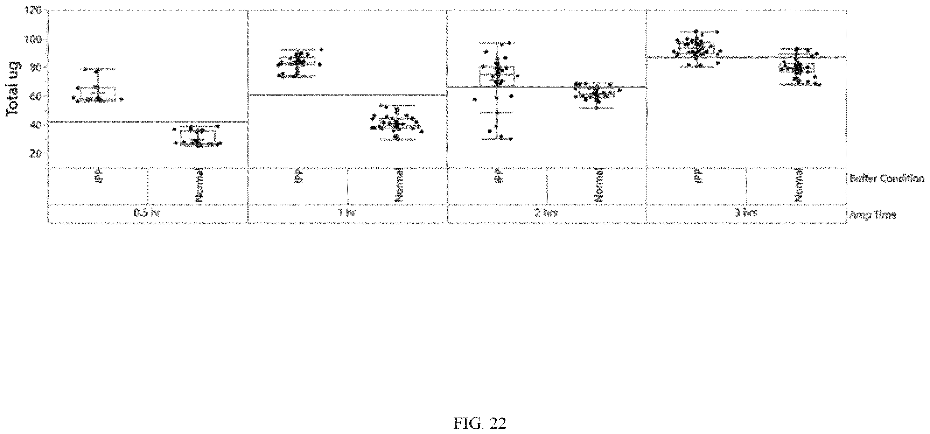

In order to decrease the generation of PPi, minimize pyrophosphorolysis and the formation of magnesium PPi complexes, iPPase was added to isothermal whole-genome amplification reactions such that the iPPase would hydrolyze PPi as it is was being generated in real-time. Whole-genome amplification reactions were incubated from 0.5 to 3 hours in either the presence or absence of iPPase (IPP) with 200 ng input DNA. FIG. 22 is a graph of total DNA yield for amplification reactions performed in the presence or absence of IPP and sampled at incubation times of 0.5 hour, 1 hour, 2 hours, and 3 hours. For reactions that did not contain iPPase, a yield of 80 µg DNA after 3 hours was observed and stayed substantially constant with time. For reactions containing iPPase, a yield of 80 µg DNA after 1 hour yield was achieved and stayed substantially constant with time. These observations were consistent with PPi generation during whole-genome amplification leading to adverse effects including increased levels of pyrohosphorolysis and magnesium PPi complex formation.

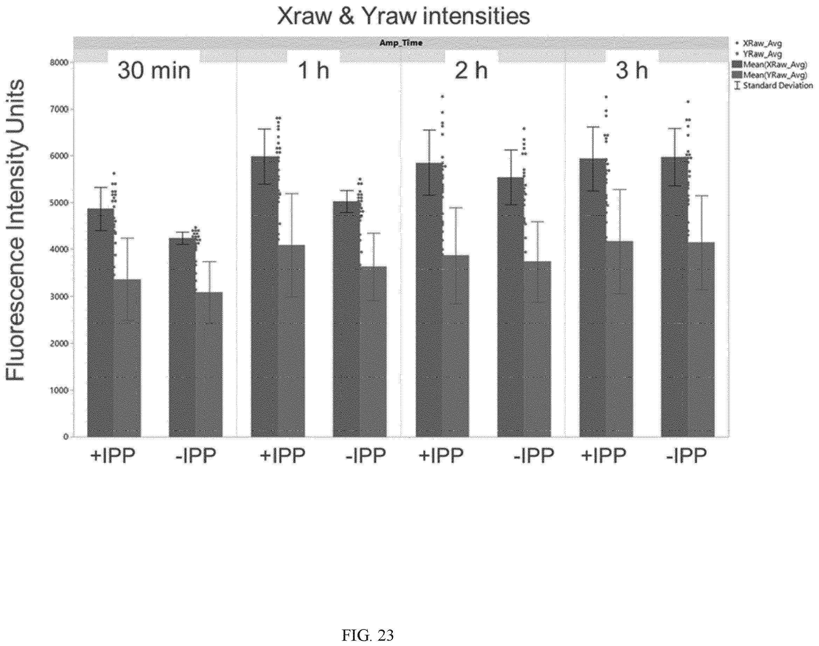

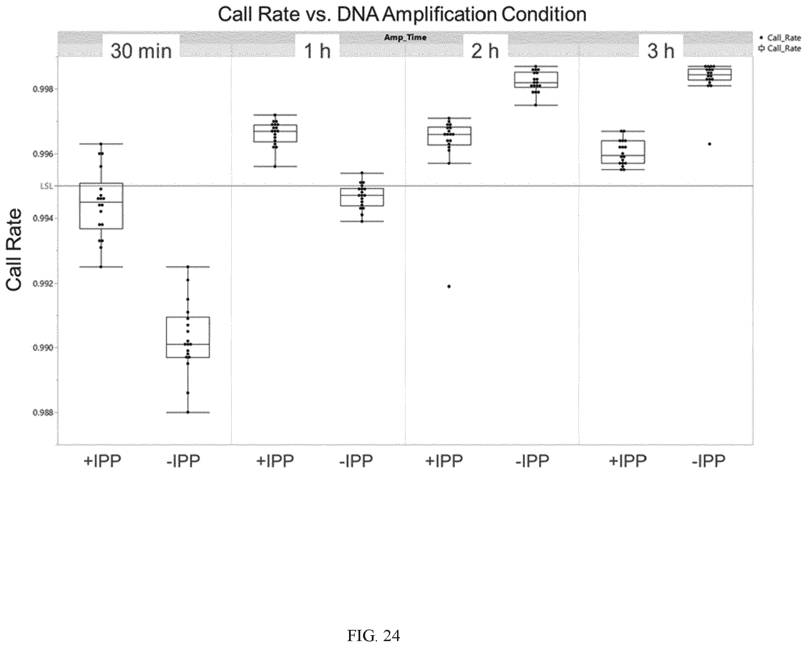

Amplification reactions were repeated with 100 ng DNA. The DNA was processed with an Infinium EX workflow to measure functional genotyping performance with 60K SNP content per sample well. The Xraw and Yraw dye intensities originating from spectrally unique dyes post staining were measured (FIG. 23). FIG. 23 depicts a graph of fluorescence intensity for amplification reactions performed with a 60K Infinium EX beadchip and in the presence or absence of iPPase (IPP) and sampled at incubation times of 0.5 hour, 1 hour, 2 hours, and 3 hours. As shown in FIG. 23, Xraw and Yraw intensity values with formulations that contained iPPase were higher at shorter amplification times (30 min and 1 h) and equal at 2 h and 3 h. Call rates for amplification reactions performed in the presence or absence of iPPase (IPP) and sampled at incubation times of 0.5 hour, 1 hour, 2 hours, and 3 hours are depicted in FIG. 24. As shown in FIG. 24, call rates, a key genotype metric, were higher for formulations that contained iPPase at 30 min and 1 h DNA amplification. Call rates exceeded the lower specification limit (LSL = 0.995) at 1 h; whereas, the formulation without iPPase required a minimum of 2 h amplification time. Thus, the presence of iPPase in amplification reactions increased reaction efficiencies substantially.

The term "comprising" as used herein is synonymous with "including," "containing," or "characterized by," and is inclusive or open-ended and does not exclude additional, unrecited elements or method steps.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope and spirit of the invention.

All references cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification, the specification is intended to supersede and/or take precedence over any such contradictory material.

## Claims

1. A method of preparing a library of nucleic acids, comprising:
(a) obtaining a substrate having a plurality of capture probes attached thereto, wherein the capture probes comprise first amplification sites and first locus specific primers;
(b) hybridizing a plurality of target nucleic acids to the first locus specific primers;
(c) hybridizing second locus specific primers to the target nucleic acids;
(d) hybridizing the second locus specific primers to extension oligonucleotides, wherein the extension oligonucleotides comprise sites complementary to second amplification sites; and
(e) extending the hybridized second locus specific primers to obtain a plurality of extended probes by:
(i) ligating the first locus specific primers to the second locus specific primers, and
(ii) extending the ligated second locus specific primers with sequences complementary to the extension oligonucleotides by polymerase extension.

2. The method of claim 1, wherein (i) comprises extending the first locus specific primers, and ligating the first locus specific primers to the second locus specific primers; and optionally, wherein:
the capture probes comprise first indexes, or first sequencing primer sites;
the extension oligonucleotides comprise sites complementary to second indexes, or complementary to second sequencing primer sites; and/or
the substrate comprises a plurality of beads or a flow cell.

3. The method of claim 1 or 2, further comprising obtaining a normalized amount of amplified target nucleic acids, comprising:
amplifying the extended probes by hybridizing extension primers to the second amplification sites, wherein the substrate comprises a normalizing amount of the capture probes, and the amount of the extension primers is equal to or greater than the normalizing amount of capture probes;
optionally, wherein: the extension primers are in solution; and/or the amplification is performed under conditions such that the normalizing amount of capture probes limits the amount of amplification products; and
optionally, further comprising sequencing the amplified target nucleic acids.

4. The method of any one of claims 1-3, wherein the first amplification sites or second amplification sites comprise a P5 sequence, a complement of a P5 sequence, P7 sequence, or a complement of a P7 sequence.

5. The method of any one of claims 1-4, wherein the first amplification sites and the extension primers are incapable of or are essentially incapable of hybridizing to one another; optionally, wherein:
(I) the first amplification sites and the extension primers are non-complementary to one another;
(II) the first amplification sites and the extension primers comprise non-complementary nucleotide sequences to one another;
(III) the first amplification sites comprise modified nucleotides that inhibit hybridization with the extension primers;
(IV) the first amplification sites and the extension primers each lack the same type of nucleotide;
(V) the first amplification sites lack at least one type of nucleotide selected from adenine (A), cytosine (C), guanine (G), and thymine (T); or
(VI) the first amplification site lacks a combination of nucleotides selected from: adenine (A) and thymine (T); or guanine (G) and cytosine (C).

6. A method of preparing a normalized nucleic acid library, comprising:
(a) obtaining a substrate comprising a plurality of capture probes and a plurality of extension primers attached thereto, wherein the capture probes comprise first amplification sites and first locus specific primers, and the extension primers are capable of hybridizing to second amplification sites, wherein the amount of the capture probes or the extension primers is a normalizing amount;
(b) hybridizing a plurality of target nucleic acids to the first locus specific primers;
(c) extending the capture probes to obtain extended probes, wherein the extended probes comprise second amplification sites;
(e) amplifying the extended probes by hybridizing the extended probes to the extension primers, to obtain a normalized amount of amplified target nucleic acids.

7. The method of claim 6, wherein: (i) the normalizing amount limits the amount of amplified target nucleic acids;
(ii) the capture probes comprise first indexes, or first sequencing primer sites;
(iii) the extension oligonucleotides comprise sites complementary to second indexes, or complementary to second sequencing primer sites;
(iv) the amplification is performed under conditions such that the normalizing amount of capture probes or the extension primers limits the amount of amplification products;
(v) the substrate comprises a plurality of beads or a flow cell;
(vi) the amplification comprises bridge amplification; and/or
(vii) the method further comprises sequencing the amplified target nucleic acids.

8. The method of claim 6, wherein extending the capture probes comprises: ligating the first locus specific primers to the second locus specific primers; or.
(i) hybridizing second locus specific primers to the target nucleic acids; and
(ii) ligating the first locus specific primers to the second locus specific primers;
optionally, wherein: the second locus specific primers comprise the second amplification sites; and/or the second locus specific primers comprise second indexes or second sequencing primer sites.

9. The method of claim 6, further comprising:
(iii) hybridizing extension oligonucleotides to the second locus specific primers; and
(iv) extending the second locus specific primers with sequences complementary to the extension oligonucleotides by polymerase extension; and
optionally, wherein:
(I) the extension oligonucleotides comprise sites complementary to the second amplification sites, complementary to second indexes, or complementary to second sequencing primer sites; and/or
(II) the target nucleic acids comprise sites complementary to the second amplification sites, and the extending comprises polymerase extension of the first locus specific primers with sequences complementary to the target nucleic acids; optionally, further comprising preparing the target nucleic acids by adding adaptors to an end of the target nucleic acids, wherein the adaptors comprise sites complementary to the second amplification sites; optionally, wherein adding adaptors comprises a tagmentation reaction.

10. A method of preparing an indexed nucleic acid library, comprising:
(a) adding Y-adaptors to a plurality of target nucleic acids, wherein the Y-adaptors are added to first and second ends of each target nucleic acid, and each Y-adaptor comprises a first strand comprising a first primer binding site and a mosaic element, and a second strand comprising a second primer binding site and a complement to the mosaic element; and
(b) hybridizing a first extension oligonucleotide to the second primer binding site, wherein the first extension oligonucleotide comprises a first index and a third primer binding site; and
(c) extending the second strand comprising the second primer binding site, thereby adding the first index to the target nucleic acids; optionally, wherein:
a 5' end of the first strand of the Y-adaptor is resistant to nuclease degradation;
a 5' end of the first strand of the Y-adaptor comprises a phosphorothioate bond between two consecutive nucleotides;
a 5' end of the second strand of the Y-adaptor is phosphorylated;
a 3' end of the first extension oligonucleotide is blocked;
a 5' end of the first extension oligonucleotide is phosphorylated; and/or
the first extension oligonucleotide is bound to a bead, optionally, the method further comprising further comprising cleaving the first extension oligonucleotide from the bead prior to (c).

11. The method of claim 10, wherein (a) comprises contacting the plurality of target nucleic acids with a plurality of transposomes, wherein each transposome comprises a Y-adaptor and a transposase; optionally, wherein:.
the transposomes comprise dimers;
the transposase comprises a Tn5 transposase; and/or
the transposomes are bound to a substrate.

12. The method of claim 10 or 11, wherein (c) comprises polymerase extension, or extension with a ligase; optionally, wherein:
the extension with a ligase comprises:
hybridizing a ligation oligonucleotide to the first extension oligonucleotide hybridized to the second primer binding site; and
ligating the ligation oligonucleotide to the second strand comprising the second primer binding site.

13. The method of any one of claims 10-12, further comprising removing the first extension oligonucleotide after step (c); optionally, wherein the removing comprises an exonuclease treatment, optionally, wherein , wherein the first extension oligonucleotide comprises uracil nucleotides, and the removing comprises degradation of the first extension oligonucleotide with a uracil-specific excision reagent (USER) enzyme.

14. The method of any one of claims 10-13, further comprising amplifying the target nucleic acids comprising the first index; optionally, wherein: the amplification comprises hybridizing amplification primers to the first primer binding sites and to the third primer binding sites; optionally, wherein the the amplification comprises a PCR or bridge amplification.

15. The method of any one of claims 10-14, further comprising adding a second index to the target nucleic acids comprising the first index; optionally, wherein adding the second index comprises:
hybridizing a second extension oligonucleotide to the third primer binding site of the target nucleic acids comprising the first index, wherein the second extension oligonucleotide comprises the second index; and
extending the second strand comprising the third primer binding site, thereby adding the second index to the target nucleic acids comprising the first index; and optionally, wherein:
a 3' end of the second extension oligonucleotide is blocked;
a 5' end of the second extension oligonucleotide is phosphorylated;
extending the second strand comprising the third primer binding site comprises polymerase extension;
extending the second strand comprising the third primer binding site comprises extension with a ligase;
the method further comprises removing the second extension oligonucleotide after extending the third primer binding site;
the method further comprises adding a third index to the target nucleic acids comprising the second index; and/or
the method further comprises adding an additional index to the target nucleic acids comprising the third index.
